(19) Europäisches Patentamt
European Patent Office
Office européen des brevets

(11) EP 1 894 920 A1

(12) EUROPEAN PATENT APPLICATION
published in accordance with Art. 153(4) EPC

(43) Date of publication:
**05.03.2008 Bulletin 2008/10**

(21) Application number: **06747178.9**

(22) Date of filing: **05.06.2006**

(51) Int Cl.:
*C07D 277/20* (2006.01) *A61K 31/426* (2006.01)
*A61K 31/428* (2006.01) *A61K 31/437* (2006.01)
*A61K 31/4439* (2006.01) *A61K 31/444* (2006.01)
*A61K 31/4709* (2006.01) *A61P 33/02* (2006.01)
*A61P 33/06* (2006.01) *C07D 277/38* (2006.01)
*C07D 277/82* (2006.01) *C07D 417/14* (2006.01)
*C07D 498/04* (2006.01) *C07D 513/04* (2006.01)

(86) International application number:
**PCT/JP2006/311198**

(87) International publication number:
**WO 2006/137258 (28.12.2006 Gazette 2006/52)**

(84) Designated Contracting States:
**CH FR GB LI**

(30) Priority: **24.06.2005 JP 2005184183**

(71) Applicants:
- **Japan Science and Technology Agency**
  **Kawaguchi-shi,**
  **Saitama 332-0012 (JP)**
- **FUJIFILM Corporation**
  **Minato-ku**
  **Tokyo 106-8620 (JP)**

(72) Inventors:
- **IHARA, Masataka**
  **Sendai-shi,**
  **Miyagi 982-0021 (JP)**
- **TAKASU, Kiyosei**
  **Sendai-shi,**
  **Miyagi 982-0036 (JP)**
- **PUDHOM, Kanitha**
  **Amphur Pakret, Nonthabnri 11120 100-1501 (TH)**
- **KITAGUCHI, Hiroshi,**
  **Minamiashigara-shi,**
  **Kanagawa 250-0193 (JP)**
- **KAWAKAMI, Masayuki,**
  **Minamiashigara-shi,**
  **Kanagawa 250-0193 (JP)**
- **SATOU, Kouzou,**
  **Minamiashigara-shi,**
  **Kanagawa 250-0193 (JP)**

(74) Representative: **Strehl Schübel-Hopf & Partner**
**Maximilianstrasse 54**
**80538 München (DE)**

# (54) PHARMACEUTICAL COMPOSITION COMPRISING AZARHODACYANINE COMPOUND AS ACTIVE INGREDIENT

(57) The object of the invention is to provide pharmaceutical composition that can be used as a therapeutic and/or prophylactic agent. Particularly, the pharmaceutical composition of the invention has significant therapeutic effect and survival benefit for the disease caused by parasitic protozoa, and selective toxicity against the causative protozoa. The pharmaceutical composition comprises a compound represented by general formula (1). Particularly, the invention relates to a composition that is an effective therapeutic/prophylactic agent for malaria, leishmania, African sleeping sickness, Chagas disease, toxoplasmosis lymphatic filariasis, babesiosis, and coccidiosis, and a novel compound contained therein.

Formula:

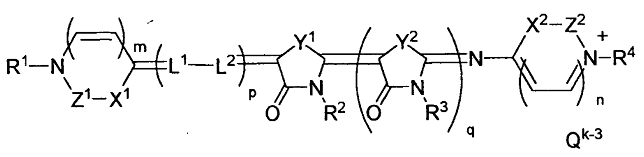

(1)



Printed by Jouve, 75001 PARIS (FR)

## Description

Field of the invention

The invention relates to a pharmaceutical composition and a novel compound as an active ingredient thereof. Particularly, the compound according to the invention is useful for treatment of diseases associated with protozoa infection such as malaria including drug-resistant malaria, leishmania, trypanosomiasis including African sleeping sickness and Chagas disease, toxoplasmosis, and cryptosporidiosis.

Background art

Many infections caused by parasitic protozoa are known especially in tropical and subtropical regions. The infections include malaria, leishmania, African sleeping sickness (African trypanosomiasis), Chagas disease (American trypanosomiasis), lymphatic filariasis, babesiosis, cryptosporidiosis, and toxoplasmosis. Some diseases infect only human, others are zoonotic infections which infect human, livestocks, and small animals. Either infection causes significant economic and social damage in the society.

Unfortunately, some of these diseases have no effective therapeutic agent so far, some causative protozoa develop drug-resistant mutant that is diffusing now, and some therapeutic agents against these diseases cause adverse effect. Effective drug with less adverse effect is highly desirable. Some of diseases develop serious symptoms which prevent a sufferer from leading ordinary social life, confines a sufferer to bed needing nursing care, or develops fatal symptoms. Drugs to be used for chemical therapy for these diseases are indispensable. However, effective vaccines against these diseases have not been developed yet, and cannot be anticipated in the foreseeable future. In this circumstance, medicine for chemical therapy which can be administered orally or by injection, or some other administration route are essential.

It is already known that a chemical compound represented by general formula (2) or (6) according to the invention as a water-soluble methine compound can be used as a material in photography, or antitumor medicine (Japanese patent application publication No. 1994-220053, 1994-293642). However, the applications do not disclose its usage as a therapeutic and/or prophylactic agent against protozoa infection.

The inventors already have disclosed that the rhodacyanines compound represented by general formula (9), which is relatively similar to the compound represented by general formula (2) of the invention, shows in vitro proliferation inhibition activity against malaria protozoa (Japanese patent application publication No. 2000-191531, 2003-034641, 2003-034642) and leishmania protozoa (Japanese patent application publication No. 2004-331545). The inventors also have disclosed that the rhodacyanines compound represented by general formula (10), which is relatively similar to the compound represented by general formula (3) of the invention, shows in vitro proliferation inhibition activity against malaria protozoa (Japanese patent application publication No. 2003-034640). However, the relationship between therapeutic effect against protozoa infections and molecular structure of the compound has not been elucidated yet. It is not known to those skilled in the art and cannot be anticipated that the azarhodacyanine compounds represented by general formulae (1) to (3), (6) and (7) according to the invention are effective as a therapeutic agent against protozoa infections.

Formula 9

Formula 10

Therapeutic effect of the compounds represented by the above general formulae (9) and (10) already known in the art was evaluated using malaria infected model animals (in vivo test). They only show 50% or less proliferation inhibition activity against malaria protozoa and survival benefit for one day at most at any dose or by any administration route. In general, therapeutic effect of an agent against malaria is evaluated in vivo based on proliferation inhibition activity and survival benefit. High proliferation inhibition activity and prolonged survival period is desirable.

The known compounds represented by general formulae (9) and (10) exert acute toxicity to a mouse. When they are intraperitoneally administered at dose of 20 mg/kg or more, all the treated mice died within 24 hours after administration.

Patent document 1
Japanese patent application publication No. 1994-220053
Patent document 2
Japanese patent application publication No. 1994-293642
Patent document 3
Japanese patent application publication No. 2000-191531
Patent document 4
Japanese patent application publication No. 2003-034641
Patent document 5

Japanese patent application publication No. 2003-034642
Patent document 6
Japanese patent application publication No. 2004-331545
Patent document 7
Japanese patent application publication No. 2003-034640

SUMMARY OF THE INVENTION

Problems to be solved by the invention

Therefore, an object of the invention is to provide a pharmaceutical composition which can be used as a therapeutic and/or prophylactic agent with high therapeutic effect on the infection caused by parasitic protozoa and selective toxicity against the causative protozoa. Particularly, an object of the invention is to provide a pharmaceutical composition which can be used as a therapeutic and/or prophylactic agent, has low toxicity to the humanuffering form infection by parasitic protozoa, and shows significant therapeutic effect when administered.

Means for solving the problem

In order to solve the above problems, the inventors measured proliferation inhibition effect of a variety of compounds against causative protozoa, and evaluated cytotoxic effect on mammalian cells as an indicator of adverse effect. As to selected compounds, therapeutic effect against malaria was evaluated by administering it in ranging dose through various administration routes using malaria infected mice as a model host, in order to select a compound which shows at least 50% of proliferation inhibition effect against malaria protozoa. As a result, it was found that the object of the invention as above can be achieved by pharmaceutical composition containing a compound represented by following general formula as an active ingredient. The invention is completed based on this finding.
Some aspects of the invention are as follows.

1. Pharmaceutical composition containing at least one of azarhodacyanine compounds represented by general formula (1) as an active ingredient:
(Formula 1)
wherein , R1 and R4 respectively represents alkyl group which may be same or different from each other, R2 and R3 respectively represents alkyl, aryl, or heterocyclic group, X1 and X2 respectively represents S, O, Se, -CR5R6- (R5 and R6 respectively represents alkyl group), -NR7- (R7 represents alkyl, aryl, or heterocyclic group), or -CR8=CR9- (R8 and R9 represents hydrogen or substituent, alternatively, R8 and R9 may bind together to form alicyclic ring, aromatic ring, or heterocyclic ring) , Y1 and Y2 respectively represents S, O, Se, or -NR8- (R8 is a alkyl, aryl, or heterocyclic group), Z1 and Z2 respectively represents an atomic group necessary for forming a five member ring or a six member ring, L1 and L2 respectively represents methine group, wherein when p>0 and L1 is a substituted methine group, L1 and R1 may bind together to form a five member ring or a six member ring, Q represents physiologically acceptable anion, k represents one integer from 0 to 2 necessary to null whole molecular charge, m and n respectively represents 0 or 1 which may be same or different from each other, p represents one integer from 0 to 2, and q represents one integer from 0 to 2.
2. Pharmaceutical composition according to claim 1, wherein the composition is used as a therapeutic and/or prophylactic agent against protozoa infection.
3. Pharmaceutical composition according to claim 2, wherein the protozoa infection is one of malaria, leishmania, African sleeping sickness, Chagas disease, toxoplasmosis, lymphatic filariasis, babesiosis, or coccidiosis.
4. Pharmaceutical composition according to claim 3, wherein the composition is used as a therapeutic and/or prophylactic agent against malaria.
5. Pharmaceutical composition containing at least one of compounds represented by general formula (2) as an active ingredient:
(Formula 2)
wherein R1 and R3 respectively represents alkyl group which may be same or different from each other, R2 represents alkyl, aryl, or heterocyclic group, X1 and X2 respectively represents S, O, Se, -CR4R5- (R4 and R5 respectively represents alkyl group), -NR6- (R6 represents alkyl, aryl, or heterocyclic group), or -CR7=CR8- (R7 and R8 represents hydrogen or substituent, alternatively, R7 and R8 may bind together to form alicyclic ring, aromatic ring, or heterocyclic ring), Z1 and Z2 respectively represents atomic group necessary for forming a five member ring or a six member ring, Q represents physiologically acceptable anion, k represents one integer from 0 to 2 necessary to null whole molecular charge, and m and n respectively represents 0 or 1 which may be same or different from each other.
6. Pharmaceutical composition according to claim 5, wherein R1 and R3 are methyl group, and R2 is ethyl group

or phenyl group.

7. Pharmaceutical composition according to claim 5 or 6, wherein the composition is used as a therapeutic and/or prophylactic agent against protozoa infection.

8. Conjugated compound represented by general formula (3): (Formula 3)

wherein, R1 and R4 respectively represents alkyl group which may be same or different from each other, R2 and R3 respectively represents alkyl, aryl, or heterocyclic group, X1 and X2 respectively represents S, O, Se, -CR5R6- (R5 and R6 respectively represents alkyl group), -NR7- (R7 represents alkyl, aryl, or heterocyclic group), or -CR8=CR9- (R8 and R9 represents hydrogen or substituent, alternatively, R8 and R9 may bind together to form alicyclic ring, aromatic ring, or heterocyclic ring), Z1 and Z2 respectively represents atomic group necessary for forming a five member ring or a six member ring, Q represents physiologically acceptable anion, k represents one integer from 0 to 2 necessary to null whole molecular charge, and m and n respectively represents 0 or 1 which may be same or different from each other.

9. A method to manufacturing the conjugated compound represented by general formula (3), the compound having a thioiminium compound represented by general formula (4) and a nitrogen containing compound represented by general formula (5) as material,

(Formula 4)

wherein, R1 and R10 respectively represents alkyl group which may be same or different from each other, R2 represents alkyl, aryl, or heterocyclic group, X1 represents S, O, Se, -CR5R6-(R5 and R6 respectively represents alkyl group), -NR7- (R7 represents alkyl, aryl, or heterocyclic group), or -CR8=CR9-(R8 and R9 represents hydrogen or substituent, alternatively, R8 and R9 may bind together to form an alicyclic ring, aromatic ring, or heterocyclic ring), Z1 represents an atomic group necessary for forming a five member ring or a six member ring, Q represents physiologically acceptable anion, k represents one integer from 0 to 2 necessary to null whole molecular charge, and m represents 0 or 1,

(Formula 5)

wherein, R4 represents alkyl group, R3 represents alkyl, aryl, or heterocyclic group, X2 represents S, O, Se, -CR5R6- (R5 and R6 respectively represents alkyl group), -NR7- (R7 represents alkyl, aryl, or heterocyclic group), or -CR8=CR9- (R8 and R9 represents hydrogen or substituent, alternatively, R8 and R9 may bind together to form alicyclic ring, aromatic ring, or heterocyclic ring), Z2 represents an atomic group necessary for forming a five member ring or a six member ring, Q represents physiologically acceptable anion, k represents one integer from 0 to 2 necessary to null whole molecular charge, and n represents 0 or 1.

10. Pharmaceutical composition containing at least one compound represented by general formula (3) as an active ingredient.

11. Pharmaceutical composition according to Claim 10, wherein R1 and R4 are methyl group.

12. Pharmaceutical composition according to claim 10 or 11,

wherein the composition is used as a therapeutic and/or prophylactic agent against protozoa infection.

13. Pharmaceutical composition containing at least one compound represented by general formula (6) as an active ingredient,

(Formula 6)

wherein , R1 and R3 respectively represents alkyl group which may be same or different from each other, R2 represents alkyl, aryl, or heterocyclic group, X1 and X2 respectively represents S, O, Se, -CR4R5- (R4 and R5 respectively represents alkyl group), -NR6- (R6 represents alkyl, aryl, or heterocyclic group), or -CR7=CR8- (R7 and R8 represent hydrogen or substituent, alternatively, R7 and R8 may bind together to form alicyclic ring, aromatic ring, or heterocyclic ring), Z1 and Z2 respectively represents an atomic group necessary for forming a five member ring or a six member ring, Q represents physiologically acceptable anion, k represents one integer from 0 to 2 necessary to null whole molecular charge, and m and n represent 0 or 1 which be may same or different from each other.

14. Pharmaceutical composition according to claim 13 wherein R1 and R3 are methyl group or ethyl group, and R2 is ethyl group.

15. Pharmaceutical composition according to claim 13 or 14, wherein the composition is used as a therapeutic and/or prophylactic agent against protozoa infection.

16. Conjugated compound represented by general formula (7): (Formula 7)

wherein, R1 and R4 respectively represents alkyl group which may be same or different from each other, R2 and R3 respectively represents alkyl, aryl, or heterocyclic group, X1 and X2 respectively represents S, O, Se, -CR5R6- (R5 and R6 respectively represents alkyl group), -NR7- (R7 represents alkyl, aryl, or heterocyclic group), or -CR8=CR9- (R8 and R9 represents hydrogen or substituent, alternatively, R8 and R9 may bind together to form alicyclic ring, aromatic ring, or heterocyclic ring), Z1 and Z2 respectively represents atomic group necessary for forming a five member ring or a six member ring, Q represents physiologically acceptable anion, k represents one integer from 0 to 2 necessary to null whole molecular charge, and m and n respectively represents 0 or 1 which

may be same or different from each other.

17. A method to manufacturing the conjugated compound represented by general formula (7), the compound having a thioiminium compound represented by general formula (8) and a nitrogen containing compound represented by general formula (5) as material,

(Formula 8)

wherein, R1 and R10 respectively represents alkyl group which may be same or different from each other, R2 represents alkyl, aryl, or heterocyclic group, X1 represents S, O, Se, -CR5R6-(R5 and R6 respectively represents alkyl group), -NR7- (R7 represents alkyl, aryl, or heterocyclic group), or -CR8=CR9-(R8 and R9 represents hydrogen or substituent, alternatively, R8 and R9 may bind together to form an alicyclic ring, aromatic ring, or heterocyclic ring), Z1 represents an atomic group necessary for forming a five member ring or a six member ring, Q represents physiologically acceptable anion, k represents one integer from 0 to 2 necessary to null whole molecular charge, and m represents 0 or 1,

(Formula 5)

wherein, R4 represents alkyl group, R3 represents alkyl, aryl, or heterocyclic group, X2 represents S, O, Se, -CR5R6- (R5 and R6 respectively represents alkyl group), -NR7- (R7 represents alkyl, aryl, or heterocyclic group), or -CR8=CR9- (R8 and R9 represents hydrogen or substituent, alternatively, R8 and R9 may bind together to form alicyclic ring, aromatic ring, or heterocyclic ring), Z2 represents an atomic group necessary for forming a five member ring or a six member ring, Q represents physiologically acceptable anion, k represents one integer from 0 to 2 necessary to null whole molecular charge, and n represents 0 or 1.

18. Pharmaceutical composition containing at least one compound represented by general formula (7) as an active ingredient.

19. Pharmaceutical composition according to Claim 18, wherein R1 and R4 are methyl group.

20. Pharmaceutical composition according to claim 18 or 19,

wherein the composition is used as a therapeutic and/or prophylactic agent against protozoa infection.

The compound contained as an active ingredient in the pharmaceutical composition according to the invention shows proliferation inhibition effect at low-dose against parasite protozoa, and shows no cytotoxic effects on mammalian cells at dose higher than the dose having proliferation inhibition effect (namely, selective toxicity index is high). In an in vivo treatment study using malaria infected mice, it is confirmed that the compound showed significantly higher recovery rate and prolonged survival compared to conventional compounds known in the art. Furthermore, it showed extremely low acute toxicity. Therefore, the compound is proved to be an effective therapeutic agent against malaria with improved adverse effect profile.

## DETAILED DESCRIPTION OF THE INVENTION

Next, the pharmaceutical composition according to the invention will be described in detail. In this specification, "the compound of the invention" includes all compounds according to the invention, represented by general formulae (1), (2), (3), (6) and (7). The compounds represented by general formulae (1) encompass compounds represented by general formulae (2), (3), (6) and (7) . The compounds represented by general formula (3) and (7) are a novel compound that has not been known yet in the art. As described in the synthesis of compound in the present specification, the above novel compounds may be prepared from the above materials by any method known to those skilled in the art.

Preferable alkyl group in the general formula representing the compound of the invention includes alkyl group having 1 to 12 carbons, more preferably 1 to 6 carbons, and the alkyl group may be in linear, branched, or cyclic form. Particularly alkyl group includes methyl, ethyl, and butyl group. Preferable aryl group in the general formula includes aryl having 5 to 15 carbons, more preferably 6 to 10 carbons. Particularly aryl group includes phenyl group, tolyl group and p-chlorophenyl group.

Preferable heterocyclic group in the general formula representing the compound of the invention is 5 to 8 member group, more preferably, 5 or 6 member group. Hetero atom includes atoms of nitrogen, oxygen, sulfur, selenium, tellurium, and phosphorous. Preferably they are atoms of nitrogen, oxygen, sulfur or selenium. Particularly heterocyclic group includes pyrrole ring, furan ring, piperidine ring, morpholine ring, piperazine ring, pyridine ring, and pyrrolidine ring. The heterocyclic group may be substituted such as dihydropirrole ring, or tetrahydropyridine ring.

The alicyclic ring in the general formula representing the compound of the invention includes those known in the art, including cyclopentene ring and cyclohexane ring.

Aromatic ring in the general formula representing the compound of the invention includes those known in the art, including benzene ring and naphthalene ring.

R7, R8 and R9 in the general formula representing the compound of the invention are substituents well known in the art. Preferable substituent includes alkyl group having 1 to 6 carbons such as methyl group, ethyl group, propyl group, and isopropyl group; alkenyl group having 2 to 6 carbons; alkynyl group having 2 to 6 carbons; alkoxy group

having 1 to 6 carbons; aryloxy group having 6 to 8 carbons; aryl group having 6 to 8 carbons; aromatic group including phenyl group and naphthyl group; substituent
amino group such as amino group, dialkylamino group, acylamino group, and sulfonylamino group; hydroxyl group; carbamoyl group; sulfamoyl group; acyloxy group having 2 to 6 carbons; carboxyl group; the alkoxycarbonyl group having 2 to 6 carbons; aminocarbonyl group; nitrile group; sulfonic acid group; nitro group; chloro group; fluoro group and bromo group. Alternatively, alkyl group, aryl group and other cyclic group as above in the general formula representing the compound of the invention may be substituted by these substituents.

In the above compound, Q is necessary for charge equilibrium. The term "physiologically acceptable anion" in the description of Q means that Q is an ion which is no toxic to the recipient when the compound is administered to the recipient and which makes the compound soluble in water. Preferable physiologically acceptable anion represented by Q includes, halogen ions such as chlorine ion, bromine ion, and iodine ion; sulfonate ion including fatty acid and aromatic sulfonate ion such as methansulfonate ion, trifluoromethanesulfonate ion, p-toluenesulfonate ion, naphthalenesulfonate ion, 2-hydroxyethansulfonate ion; sulfamate ion such as cyclohexanesulfamate ion; sulfate ion such as methylsulfate ion and ethylsulfate ion; hydrogensulfate ion; borate ion; alkyl and dialkyll phosphate ion such as diethylphosphate ion and methylhydrogen phosphate ion,; pyrophosphate ion such as trimethylpyrophosphate ion; carboxylate ion (carboxylate ion having carboxy group and hydroxyl group substituted can be conveniently used); carbonate ion; hydrogen carbonate ion; perchlorate ion; and hydroxyl ion. Most preferable physiologically acceptable anion Q includes chlorine ion, acetate ion, propionate ion, valerate ion, citrate ion, maleate ion, fumarate ion, lactate ion, succinate ion, tartarate ion, benzoate ion, perchlorate ion, and hydroxyl ion.

Preferable 5 member ring or 6 member ring formed by Z1 and Z2 in the general formula representing the compound of the invention includes, thiazole ring (thiazole, 4-methylthiazole, 4-phenylthiazole, 4,5-diphenylthiazole, 4,5-dimethylthiazole); benzothiazole ring (benzothiazole, 5-methylbenzothiazole, 5-phenylbenzothiazole, 5-methoxybenzothiazole, 4-fluorobenzothiazole, 5, 6-dioxymethylenebenzothiazole, 5-nitrobenzothiazole, 5-trifluoromethylbenzothiazole, 5-methoxycarbonylbenzothiazole, 6-hydroxybenzothiazole, 5-cyanobenzothiazole, 5-iodobenzothiazole); naphtothiazole ring (α-naphtothiazole, β-naphtothiazole, γ-naphtothiazole, 5-methoxy-β-naphtothiazole, 8-methoxy-α-naphtothiazole, 6-methoxy-8-acetoxy-β-naphtothiazole, 8,9-dihydroxy-β-naphtothiazole); oxazole ring (4-methyloxazol, 4-phenyloxazol, 4,5-diphenyloxazol, 4-phenoxyloxazol); benzoxazole ring (benzoxazole, 5-chlorobenzoxazole, 5, 6-dimethylbenzoxazole, 6-hydroxybenzoxazole, 5-phenylbenzoxazole); naphtoxazole ring (α-naphtoxazole, β-naphtoxazole, γ-naphtoxazole); selenazole ring (for example, 4-methylselenazole, 4-phenylselenazole); benzselezole or benzselenazole ring (benzselenazole, 5-chlorobenzselenazole, 5,6-dimethylbenzselenazole, 6-hydroxybenzselenazole, 5-phenylbenzselenazole); thyazoline ring (thyazoline, 4,4-dimethylthyazoline); 2-pyridine ring (2-pyridine, 5-methyl-2-pyridine, 5-methoxy-2-pyridine, 4-chloro-2-pyridine, 5-carbamoyl-2-pyridine, 5-methoxycarbonyl-2-pyridine, 4-acetylamino-2-pyridine, 6-methylthio-2-pyridine, 6-methyl-2-pyridine); 4-pyridine ring (4-pyridine, 3-methoxy-4-pyridine, 3,5-dimethyl-4-pyridine, 3-chloro-4-pyridine, 3-methyl-4-pyridine); 2-quinoline ring (2-quinoline, 6-methyl-3-quinoline, 6-chloro-2-quinoline, 6-ethoxy-2-quinoline, 6-hydroxy-2-quinoline, 6-nitro-2-quinoline, 6-acetylamino-2-quinoline, 8-fluoro-2-quinoline); 4-quinoline ring (4-quinoline, 6-methoxy-4-quinoline, 6-acetylamino-4-quinoline, 8-chloro-4-quinoline, 8-trifluoromethyl-4-quinoline); 1-isoquinoline ring (1-isoquinoline, 6-methoxy-1-isoquinoline, 6-acetylamino-1-isoquinoline, 6-chloro-1-isoquinoline); 3,3-dialkylindolenine ring (3,3-dimethylindolenine, 3,3,7-trimethylindolenine, 5-chloro-3,3-dimethylindolenine, 5-ethoxycarbonyl-3,3-dimethylindolenine, 5-nitro-3,3-dimethylindolenine, 3,3-dimethyl-4,5-phenyleneindolenine, 3,3-dimethyl-6,7-phenyleneindolenine, 5-acetylamino-3,3,-diethylindolenine, 5-diethylamino-3,3-dipropylindolenine, 5-benzoylamino-3-ethyl-3-methylindolenine); imidazole ring (imidazole, 1-methyl-4-phenylimidazole, 1-benzil-4,5-dimethylimidazole); benzimidazole ring (benzimidazole, 1-methylbenzimidazole, 1-methyl-5-trifluoromethylbenzimidazole, 1-ethyl-5-chlorobenzimidazole, 1-phenyl-5-methoxycarbonylbenzimidazole, 1-6thyl-5-dimethylaminobenzimidazole); naphtoimidazole ring (1-methyl-α-naphtoimidazole, 1-methyl-5-methoxy-β-naphtoimidazole).

Typical compound used in the invention includes the following compounds. However, the invention is not limited to these compounds. Compounds represented by B-1 to B-42 and D-1 and D-2 are novel compounds. Therefore, the invention also relates to these novel compounds.

Chemical structures of the compounds of the invention are as follows. Compounds A-1 to A-22 are compounds represented by general formula (2). Compounds B-1 to B-42 are compounds represented by general formula (3) according to the invention. Compounds C-1 to C-12 are compounds represented by general formula (6) according to the invention. Compounds D-1 and D-2 are compounds represented by general formula (7) according to the invention.

A-1

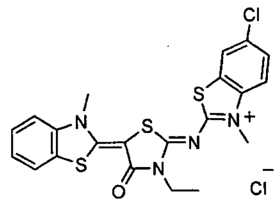

A-2

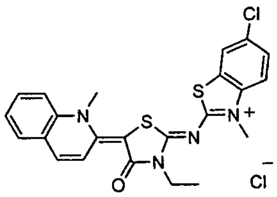

A-3

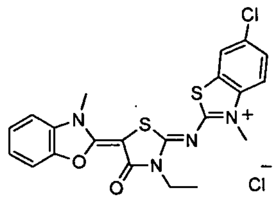

A-4

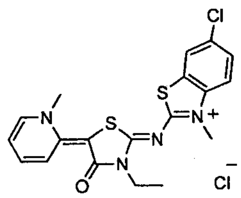

A-5

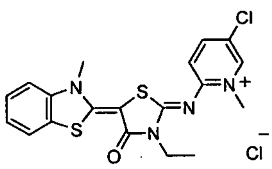

A-6

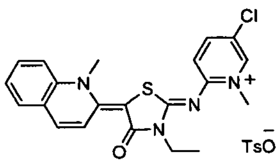

A-9

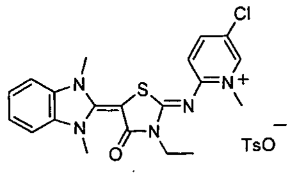

A-10

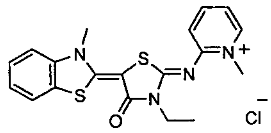

A-11

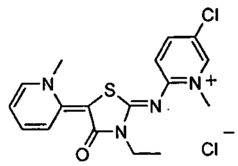

A-12

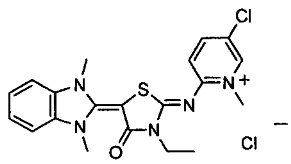

A-13

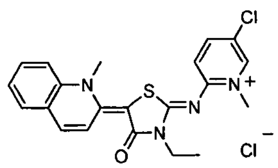

A-14

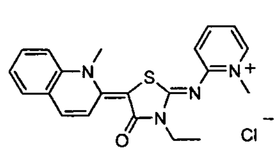

A-7 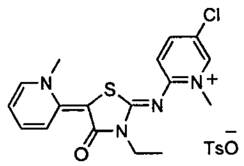

A-15 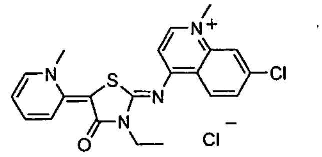

A-8 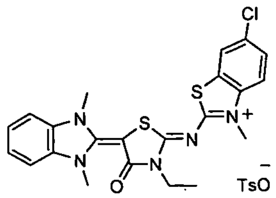

A-16 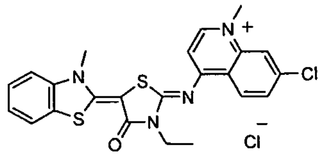

A-17 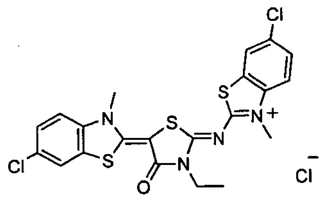

A-18 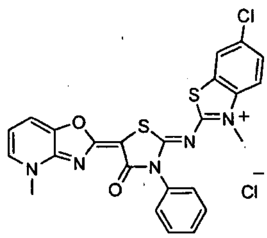

A-19 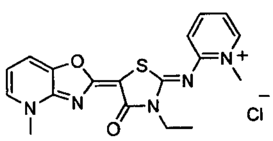

B-1 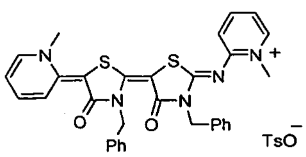

B-8 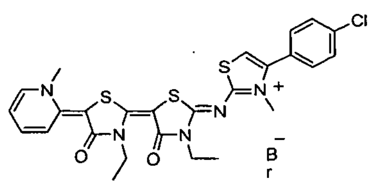

B-2

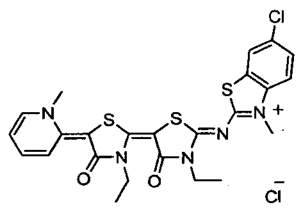

B-3

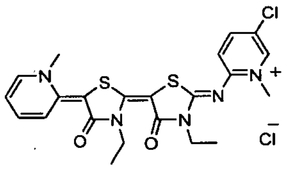

B-4

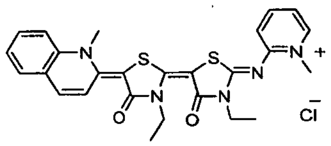

B-5

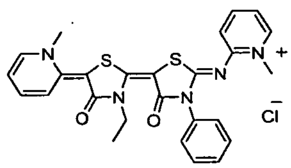

B-6

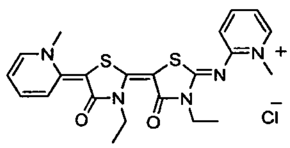

B-7

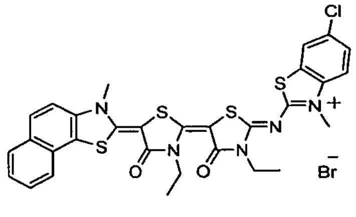

B-9

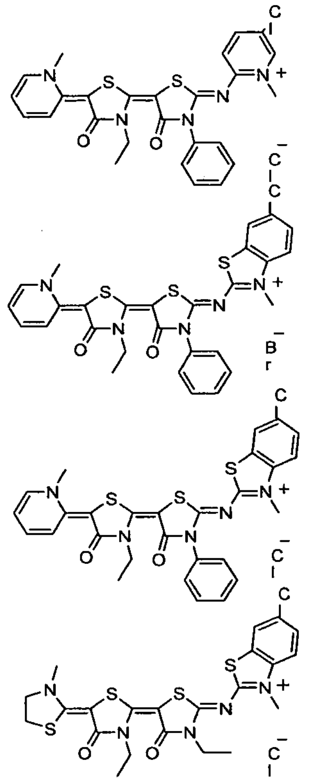

B-10

B-11

B-12

B-13

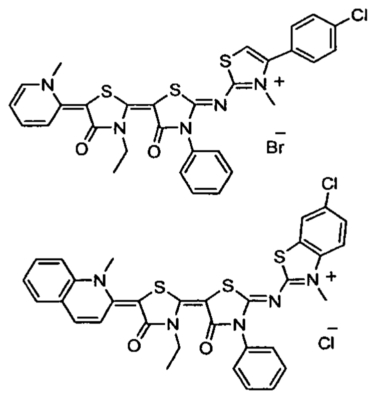

B-14

B-15

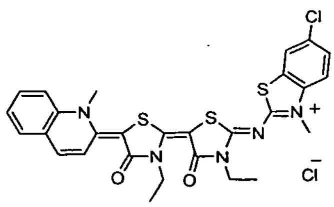

B-16 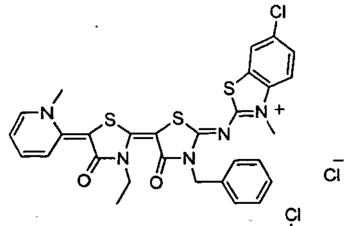

B-24 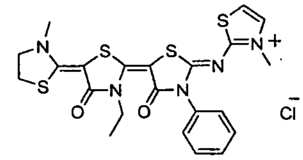

B-17 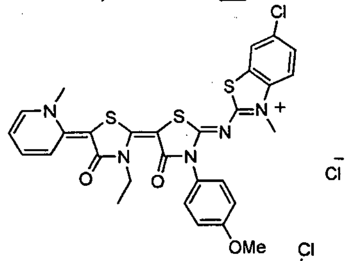

B-25 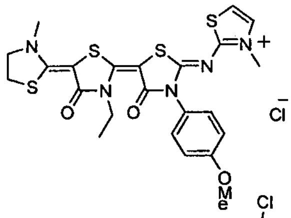

B-18 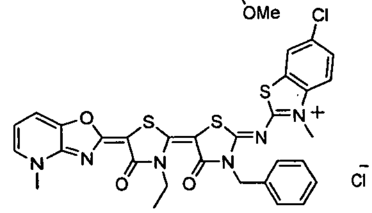

B-26 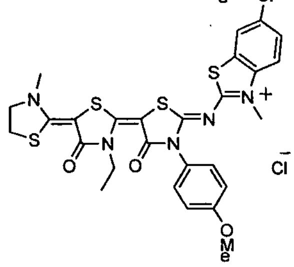

B-19 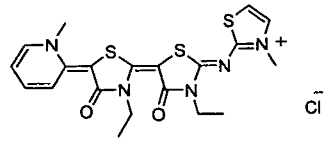

B-27 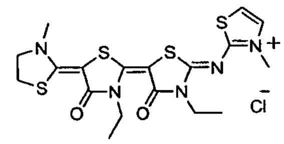

B-20 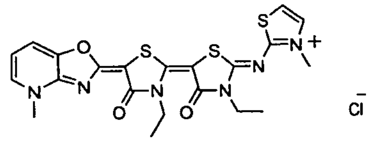

B-28 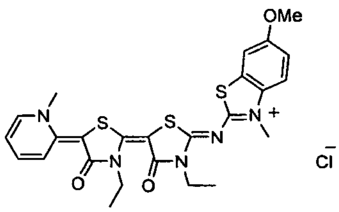

B-21 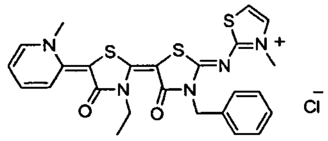

B-29 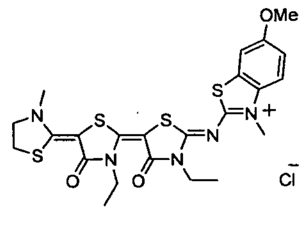

B-22 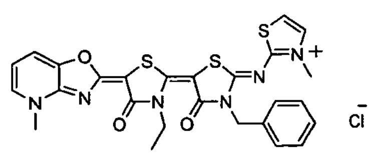

B-30 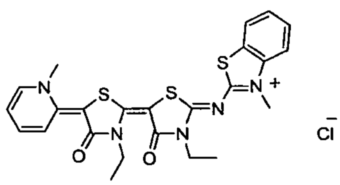

B-23 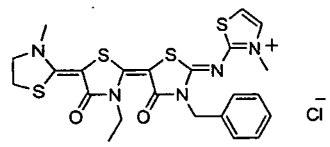

B-31 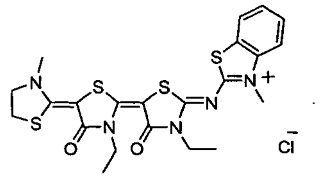

B-32 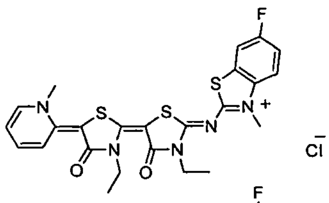

B-40 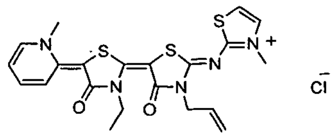

B-33 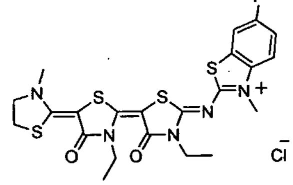

B-41 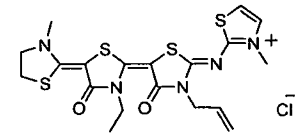

B-34 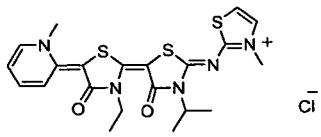

B-35 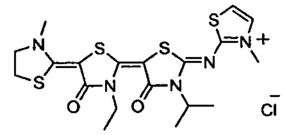

B-36 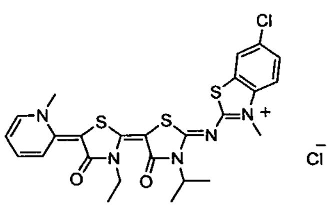

B-37 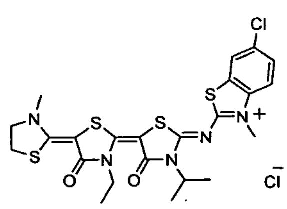

B-38

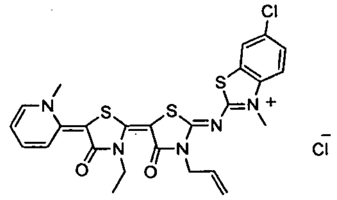

B-39

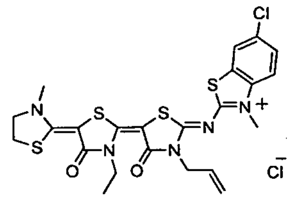

C-1

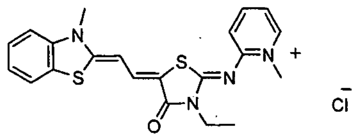

C-2

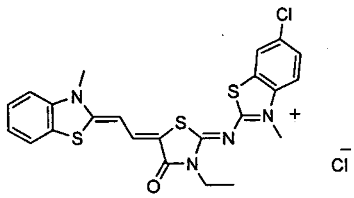

C-3

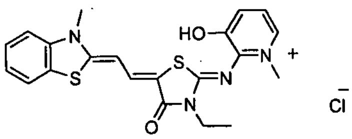

C-4

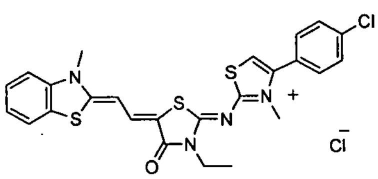

C-5 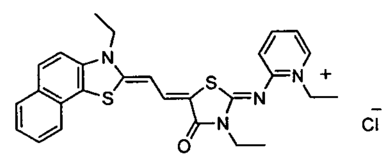

C-6 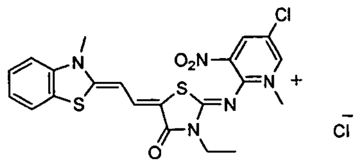

**A-20** 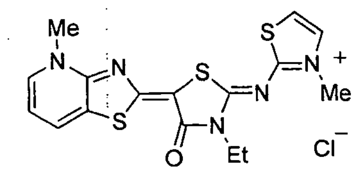

**C-9** 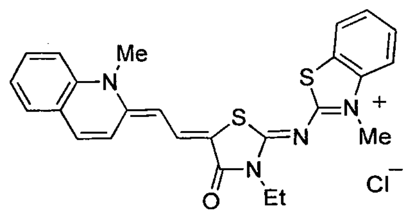

**A-21** 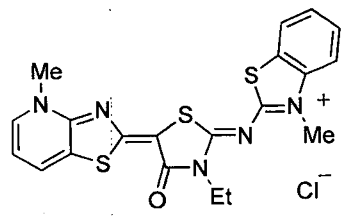

**C-10** 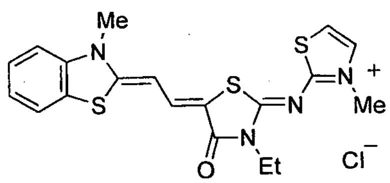

**A-22** 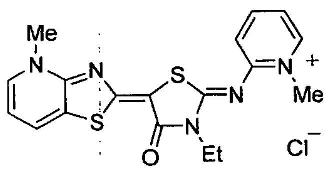

**C-11** 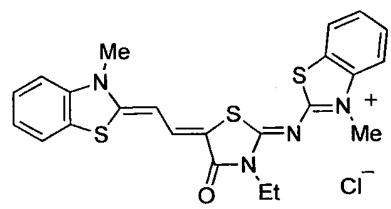

**B-42** 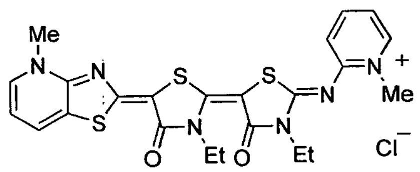

**C-12** 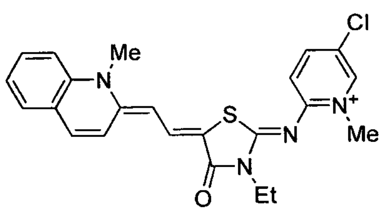

C-7 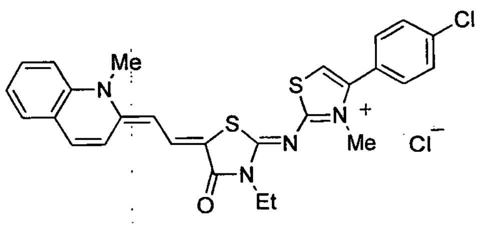 D-1 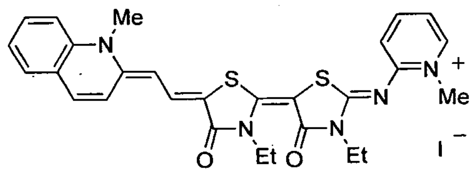

C-8 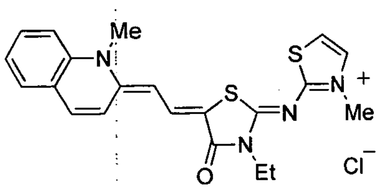 D-2 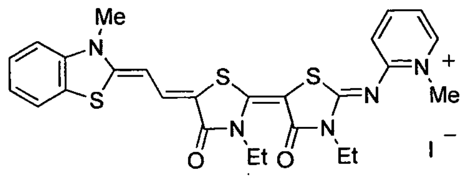

Compounds represented by general formulae (9) and (10) are controls to those of the invention represented by general formulae (1) to (3), (6) and (7). Compounds S-1 to S-4 are compounds represented by general formula (9). Compounds S-5 to S-6 are compounds represented by general formula (10).

S-1 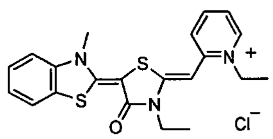

S-2 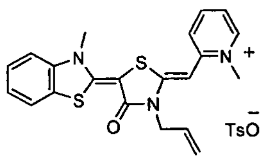

S-3 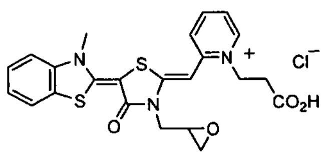

S-4 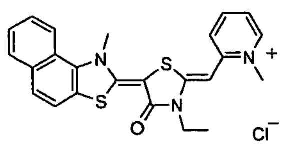

S-5 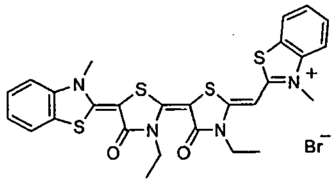

S-6 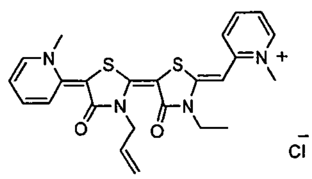

The pharmaceutical composition containing the above compounds can be used as an effective therapeutic and prophylactic agent against malaria, African trypanosomiasis (African sleeping sickness), American trypanosomiasis (Chagas disease), leishmania, babesiosis, lymphatic filariasis, toxoplasmosis (opportunistic infection caused by HIV infection), cryptospolidiosis (tropical diarrhea), and other various types of infections caused by parasitic protozoa.

The pharmaceutical composition of the invention may contain one or more compounds of the invention as active ingredients. Furthermore, the composition may be used in combination with other therapeutic agent known in the art including conventional anti-protozoa infection drugs. Preferable anti-protozoa infection drugs include chloroquine, mefloquine, artemisinin, atovaquone and pyrimethamine (therapeutic drug for malaria); suramin, pentamidine, melarsoprol, and ascofuranone (therapeutic drug for African sleeping sickness), benznidazole (therapeutic drug for chagas disease), pentostam, amphotericin B, miltefosine, and fluconazole (therapeutic drug for leishmaniasis).

Preferable pharmaceutical carrier or diluent which can be used for pharmaceutical composition of the invention in combination with the compound represented by general formula (1) includes; sodium chloride; magnesium chloride; zinc chloride; glucose; saccharose; lactose; ethyl alcohol; glycerin; mannitol; sorbitol; pentaerythritol; diethylene glycol, propylene glycol, dipropyrene glycol, polyethylene glycol 400, other polyethylene glycols; monotriglyceride, ditriglyceride, or triglyceride of fatty acid such as glyceryl trilaurate and glyceryl distearate; pectin; starch; arginine acid; xylose; talc; lycopodium; oil and fat such as olive oil, peanut oil, castor oil, corn oil, safflower oil, a wheat germ oil, sesame oil, cotton oil, sunflower seed oil and cod liver oil; gelatin; lecithin; silica; cellulose; cellulose derivatives such as methyl hydroxypropylcellulose, methylcellulose and hydroxyethyl cellulose; fatty acid salt having 12 to 22 carbon atoms such as calcium stearate, calcium laurate, magnesium oleatete, calcium palmitate, calcium behenate, magnesium stearate; cyclodextrin (α-cyclodextrin, β-cyclodextrin, γ-cyclodextrin, hydroxyethyl-β-cyclodextrin, hydroxypropyl-β-cyclodextrin, dihydroxypropyl-β-cyclodextrin, carboxymethylethyl-β-cyclodextrin, cycloawaodorin, dimethyl-β-cyclodextrin); emulsifier (saturated and unsaturated fatty acid having 2 to 22, particularly 10 to 18 carbon atoms; ester with monovalent aliphatic alcohol or multivalent alcohol having 1 to 20 carbon atoms such as glycol, glycerin, diethylene glycol, pentaerythritol, ethyl alcohol butyl alcohol, octadecyl alcohol; and silicone such as dimethylpolysiloxane. Other additional carrier which is conventionally used for pharmaceutical composition and known to those skilled in the art can be used for pharmaceutical composition of the invention, as well.

Pharmaceutically effective dose and administration method/route of the composition of the invention can be selected by those skilled in the art as appropriately depending on the species of the causative parasitic protozoa, site of the parasite, severity of the illness, therapeutic strategy, and patient's condition (age, weight, sex, general condition, and genetic ethnicity). Generally, 1 to 10,000 mg/day/70 kg, more generally 50 to 2000 mg/day/70 kg of the compound of the invention can be administered.

The pharmaceutical composition of the invention can be formulated into any form known to those skilled in the art depending on the administration method/route, and can be administrated accordingly. For example, the composition can be formulated into liquid, tablet, colloid medicine. Liquid medicine can be dissolved into 5% glucose aqueous solution, alternatively in combination with a carrier or dilution as above, and used for intravenous, intraperitoneal, or subcutaneous injection. Tablet medicine can be administered orally, and colloid medicine can be applied to skin. Appropriate amount of the compound represented by general formula (1) can be contained in the medicine depending on the purpose, subject who takes the medicine, and formulation of the pharmaceutical composition of the invention. Generally, the medicine may contain 1 mg to 10,000 mg, more preferably 10 mg to 3,000 mg compound of the invention.

EXAMPLE

Next, an example will be described for detailed illustration of the invention. Particularly, in order to clearly show the efficacy of the compound represented by general formulae (1) to (3), (6) and (7)used in the composition of the invention, proliferation inhibition activity was evaluated in in vitro screening test using malaria protozoa, leishmania protozoa, African trypanosoma protozoa, and American trypanosoma protozoa, and therapeutic effect was evaluated in in vivo study using malaria infected mouse. However, the technical field covered by the invention is not limited to these examples.

Experiment

1. Culture of chloroquine-resistant tropical malaria protozoa In this experiment, Plasmodium Falciparum k1 strain protozoa were used. The medium used in the experiment was filter-sterilized RPMI-1640 medium. To the medium humanerum was added to achieve 5% concentration. The protozoa was cultured under condition of $O_2$ concentration 3%, $CO_2$ concentration 4%, $N_2$ concentration 93%, and temperature 37°C.

2. Chloroquine-resistant tropical malaria protozoa proliferation inhibition screening test

Either the compound of the invention, control compounds (S-1 and S-3), and positive controls (Chloroquine) was dissolved into DMSO, to prepare test solutions having predefined concentration. The cultured malaria infected red blood cells were collected by centrifugation, diluted with non-infected red blood cells, to achieve initial infection rate 0.15%. The hematocrit was 2.5%.
To each well in a 96-well culture plate, 200 μL malaria infected culture was added, and either test solution containing test compound at predefined concentration or DMSO having no test compound was added. Each test solution was prepared in duplicate. After 48 hours culture under 37°C, hypoxanthine labeled with radioactive tritium ($^3H$) of 0.5μCi was added to each well. After culturing under same condition for 24 hours, the product was collected on a glass fiber filter and washed. The intensity of radiation was measured by Beta plate liquid scintillation counter (Wallac Inc.), to determine malaria protozoa infection rate in test compound samples and control samples.
Using the malaria protozoa infection rate, the proliferation inhibition rate was calculated according to the following equation to determine 50% proliferation inhibition concentration ($EC_{50}$).

$$\text{Proliferation inhibition rate (\%)} = \{1 - (b - a)/(c - a)\} \times 100$$

a: initial infection rate
b: infection rate of test compound solution
c: infection rate of the control

3. Rat L6 cell proliferation inhibition test
Rat-derived L6 cells (rat skeletal myoblast cell) were used for this test. The medium was prepared as follows: to RPMI 1640 medium, L-glutamine (200 mM) and fetal bovine serum was added to achieve concentration of 1%, 10%. The sample was cultured under $CO_2$ concentration 5% and temperature 37°C. Either the composition of the invention or control was dissolved into DMSO, to prepare test solutions having predefined concentration. The samples were precultured until logarithmic growth phase was reached. Then the culture was transferred to each well in a 96-well culture plate, and either test solution containing test compound at predefined concentration or DSMO without compound was added. The test solution was duplicated. The plate was cultured in an incubator for 72 hours, to examine proliferation inhibition activity. The activity was examined as follows. Alamar Blue water solution 10μL was added to each well, and culture was continued for 2 hours. Next, the culture plate was mounted on a fluorescence microplate reader (Spectramax Gemeni XS; Molecular Devices Corporation, U.S.), and irradiated with excitation wavelength 536nm to determine fluorescence at 588nm. The survival rate of L6 cells in the test solution sample and control sample was calculated.
Using the survival rate thus obtained, the proliferation inhibition rate against L6 cells was calculated according to the following equation to determine 50% proliferation inhibition concentration ($EC_{50}$).

$$\text{Proliferation inhibition rate (\%)} = \{(C - A)/(B - A)\} \times 100$$

A: Initial cell count
B: Cell count in control after 3 days
C: Cell count in test solution after 3 days

4. Determination of effect on Chloroquine-resistant malaria protozoa
Anti-malaria effect of the sample was evaluated based on the $EC_{50}$ value of the sample for Chloroquine-resistant tropical malaria protozoa and rat L6 cells. Chemotherapeutic index, which is used as an indicator of selective toxicity against Chloroquine-resistant malaria protozoa, was calculated according to the following equation to determine effect of the test compound. The higher the selective toxicity value, the lower the adverse effect risk (side effects).
Chemotherapeutic index = ($EC_{50}$ value of sample against rat L6 cells) / ($EC_{50}$ value of sample against Chloroquine-resistant malaria protozoa)

The $EC_{50}$ values and selective toxicity index of the compound of the invention and positive control against Chroloquine-resistant malaria protozoa and rat L6 cells are respectively shown in table 1.
Table 1

| Compound | $EC_{50}$ (μg/ml) | | Selective toxicity |
|---|---|---|---|
| | *P.falciparum* K1 | cytotoxicity L6 | |
| A-1 | 0.1485 | 37.93 | 255 |
| A-2 | 0.0022 | 5.5 | 2500 |
| A-9 | 0.046 | >90 | >2000 |
| A-10 | 0.0025 | 49 | 20000 |
| A-17 | 0.066 | 8.41 | 127 |
| A-20 | 0.011 | 24.33 | 2211 |
| A-21 | 0.002 | 6.01 | 3005 |
| A-22 | 0.0115 | >90 | >782 |
| B-2 | 0.006 | 26.97 | 4495 |
| B-12 | 0.0136 | 6.9 | 507 |
| B-19 | 0.0166 | 27.92 | 1628 |
| B-20 | 0.017 | 69.34 | 4079 |
| B-22 | 0.005 | >90 | > 18000 |
| B-28 | 0.007 | 5.01 | 715 |
| B-29 | 0.006 | 1.98 | 330 |
| B-30 | 0.0119 | 7.46 | 627 |
| B-32 | 0.0162 | 15.88 | 980 |
| B-33 | 0.0167 | 9.83 | 588 |
| B-35 | 0.006 | 21.78 | 3630 |
| B-36 | 0.009 | 10.49 | 1165 |

(continued)

| Compound | EC$_{50}$ (μg/ml) | | Selective toxicity |
|---|---|---|---|
| | *P.falciparum* K1 | cytotoxicity L6 | |
| B-37 | 0.009 | 1.44 | 160 |
| B-40 | 0.0012 | 27.38 | 2282 |
| C-2 | 0.02495 | 11.33 | 454 |
| C-5 | 0.049 | 1.3 | 27 |
| S-3 | 0.28 | >90 | >320 |
| Chloroquine | 0.047 | 60 | 1300 |

As clearly shown in the table, the compound of the invention showed proliferation inhibition effect equivalent or exceeding that of Chloquine (anti-malaria drug currently in clinical use) as a positive control, and that of control compound S - 3 against malaria protozoa. Furthermore, some compounds of the invention showed high selective toxicity value as an adverse effect index. Based on these findings, it was determined that the compound of the invention is an effective therapeutic agent against malaria.

5. Culture of African trypanosoma protozoa

In this experiment, bloodstream form trypomastigote of Trypanosoma brucei rhodensiense (STIB 900 strain) was used. The medium used in the experiment was prepared as follows: to filter-sterilized MEM medium, 25mM N-2-hydroxyethylpiperazine-2-ethane sulfonate (HEPES), 1g/L glucose, 1% MEM nonessential amino acid, 0.2 mM 2-mercaptoethanol, 2 mM pyruvic calcium salt, 0.1 mM hypoxanthine, and 15% heat-treated horse serum were added. The protozoa was cultured under atmosphere of $CO_2$ concentration 5% and temperature 37°C.

6. African trypanosoma protozoa proliferation inhibition screening test

Either the compound of the invention or a positive control (melarsoprol) was dissolved into dimethylsulfoxide (DMSO) to prepare test solution having predefined concentration. To each well in a 96-well culture plate, medium containing 8 x $10^3$ protozoa, and either test solution containing the test compound at predefined concentration or DMSO without compound was added. Then medium was added to achieve 100μL. The test solution was duplicated.

The culture plate was cultured in an incubator for 72 hours, and the proliferation inhibition activity was examined. The activity was determined as follows. To each well, 10 μL Alamar blue water solution was added, then the plate was again cultured for 2 hours. Then the culture plate was mounted on a fluorescence microplate reader (Spectramax Gemeni XS; Molecular Devices Corporation, U.S.), and irradiated with 536nm excitation wavelength to determine fluorescence at 588nm. The trypanosoma infection rate of the sample containing the test composition and that of the control sample was calculated.

Using the protozoa infection rate thus obtained, the proliferation inhibition rate was calculated according to the following equation to determine 50% proliferation inhibition concentration (EC$_{50}$).

$$\text{Proliferation inhibition rate (\%)} = \{1 - (b - a)/(c - a)\} \times 100$$

a: initial infection rate
b: infection rate of the test compound solution
c: infection rate of the control

4. Determination of effect on African trypanosoma protozoa Selective toxicity index, which is used as an indicator of selective toxicity against African trypanosoma protozoa, was calculated according to the following equation to determine effect of the test composition.

Selective toxicity index = ($EC_{50}$ value of sample against rat L6 cells) / ($EC_{50}$ value of sample against African trypanosoma protozoa)

The $EC_{50}$ values and selective toxicity index of the compound of the invention and positive control against African trypanosoma protozoa and rat L6 cells are respectively shown in table 2.

Table 2

| Compound | $EC_{50}$ (μg/ml) | | Selective toxicity |
|---|---|---|---|
| | *Trypanosoma brucei rhod* | cytotoxicity L6 | |
| A-1 | 0.403 | 37.93 | 94 |
| A-2 | 0.076 | 5.5 | 72 |
| A-9 | 1.7 | >90 | >53 |
| A-10 | 0.22 | 49 | 220 |
| A-17 | 0.789 | 8.41 | 10.6 |
| A-20 | 0.095 | 24.33 | 256 |
| A-21 | 0.029 | 6.01 | 207 |
| B-2 | 0.1045 | 26.97 | 258 |
| B-12 | 0.138 | 6.9 | 50 |
| B-22 | 0.068 | 790 | 1323 |
| B-28 | 0.042 | 5.01 | 119 |
| B-29 | 0.023 | 1.98 | 86 |
| B-30 | 0.0625 | 7.46 | 119 |
| B-32 | 0.1585 | 15.88 | 100 |
| B-33 | 0.1485 | 9.83 | 66 |
| B-36 | 0.123 | 10.49 | 85 |
| B-37 | 0.052 | 1.44 | 19 |
| B-39 | 0.141 | 3.72 | 26 |
| C-5 | 0.19 | 1.3 | 6.8 |
| S-3 | 6.1 | >90 | >15 |
| Melarsoprol | 0.0024 | 3.1 | 1300 |

As clearly shown in the table, the compound of the invention showed proliferation inhibition effect equivalent or exceeding that of melarsoprol (anti-African trypanosoma drug currently in clinical use) as a positive control, and that of control compound S - 3 against African trypanosoma protozoa. Based on these findings, it was determined that the compound of the invention is an effective therapeutic agent against African trypanosoma (African sleeping sickness).

8. Culture of American trypanosoma protozoa

In this experiment, amastigote and trypomastigote of Trypanosoma cruzi (Tulahuen C2C4 strain) infected to rat L6 cells were used. The medium used in the experiment was prepared as follows: to RPMI 1640 medium containing L6 cells, L-glutamine (200 mM) and fetal bovine serum was added to achieve concentration of 1% and 10%, respectively. The culture was conducted under $CO_2$ concentration 5% and temperature 37°C.

9. American trypanosoma protozoa proliferation inhibition screening test

Either the compound of the invention or a positive control (benznidazole) was dissolved into DMSO to prepare test solution having predefined concentration. To each well in a 96-well culture plate, medium containing 5 x $10^3$ protozoa was added and precultured for 48 hours. After replacing the medium, either the test solution containing compound of the invention in predefined concentration or DMSO without compound was added. The test solution was duplicated. The plate was cultured in an incubator for 96 hours, and the proliferation inhibition activity was examined. The activity was determined as follows. To each well, 50 μL CPRG/Nonidet was added, then left for 2 to 6 hours. Then the culture plate was mounted on an absorption microplate reader to determine absorption at 540nm. The trypanosoma infection rate of the sample containing the test compound and that of the control sample was calculated.

Using the protozoa infection rate thus obtained, the proliferation inhibition rate was calculated according to the following equation to determine 50% proliferation inhibition concentration ($EC_{50}$).

$$\text{Proliferation inhibition rate (\%)} = \{1 - (b - a)/(c - a)\} \times 100$$

a: initial infection rate
b: infection rate of the test compound solution
c: infection rate of the control

10. Determination of effect on American trypanosoma protozoa Selective toxicity index, which is used as an indicator of selective toxicity against American trypanosoma protozoa, was calculated according to the following equation to determine effect of the compound of the invention.

$$\text{Selective toxicity index} = (EC_{50} \text{ value of sample against rat L6 cells}) / (EC_{50} \text{ value of sample against American trypanosoma protozoa})$$

The $EC_{50}$ values and selective toxicity index of the compound of the invention and positive control against American trypanosoma protozoa and rat L6 cells are respectively shown in table 3.

Table 3

| Compound | $EC_{50}$ (μg/ml) | | Selective toxicity |
|---|---|---|---|
| | *Trypanosoma cruzi* | cytotoxicity L6 | |
| A-2 | 0.31 | 5.5 | 18 |
| A-20 | 0.763 | 24.33 | 32 |
| A-21 | 0.145 | 6.01 | 41 |
| B-12 | 0.98 | 6.9 | 7 |
| B-28 | 0.314 | 5.01 | 16 |
| B-29 | 0.091 | 1.98 | 21 |
| B-30 | 0.74 | 7.46 | 10 |
| B-33 | 0.66 | 9.83 | 15 |
| B-37 | 0.479 | 1.44 | 3 |
| B-39 | 0.141 | 3.72 | 26 |
| C-5 | 0.64 | 1.3 | 2 |
| S-3 | >30 | >90 | - |
| Benznidazole | 0.87 | - | - |

As clearly shown in the table, the compound of the invention showed proliferation inhibition effect equivalent or exceeding that of benznidazole (anti-American trypanosoma (Chagas' disease) drug currently in clinical use) as a positive control, and that of control compound S - 3 against American trypanosoma protozoa. Based on these findings, it was determined that the compound of the invention is an effective therapeutic agent against American trypanosoma (Chagas' disease).

11. Culture of leishmania protozoa

In this experiment, Leishmania donovani (MHOM/ET/67/L82 strain) was used. The protozoa were successively cultured in Syrian Golden hamsters to obtain amastigote. The medium used in the experiment was SM medium having heat-treated fetal bovine serum 10% added, and pH prepared to 5.4. The culture was conducted under $CO_2$ concentration 5% and temperature 37°C.

12. Leishmania protozoa proliferation inhibition screening test

Either the compound of the invention or a positive control (miltefosine) was dissolved into DMSO to prepare test solution having predefined concentration. To each well of a 96-well culture plate, medium containing predeteremined number of protozoa was added and the culture was pre-treated. The concentration of amastigote was determined by CASY cell analyze system (Scharfe GMBH Co. , Germany). Subsequently, either test solution containing compound of the invention in predefined concentration or DMSO without compound was added. The test solution was duplicated.

The plate was cultured in an incubator for 72 hours, and the proliferation inhibition activity was examined. The activity was determined as follows. To each well, 10 μL Alamar blue aqueous solution was added, then cultured again for 2 hours. Then the culture plate was mounted on a fluorescence microplate reader (Spectramax Gemeni XS; Molecular Devices Corporation, U.S), and irradiated 536nm excitation wavelength to determine absorption at 588nm. The leishmania infection rate of the sample containing the test compound and that of the control sample was calculated.

Using the protozoa infection rate thus obtained, the proliferation inhibition rate was calculated according to the following equation to determine 50% proliferation inhibition concentration ($EC_{50}$).

$$\text{Proliferation inhibition rate (\%)} = \{1 - (b - a)/(c - a)\} \times 100$$

a: initial infection rate
b: infection rate of test compound solution
c: infection rate of the control

13. Determination of effect on leishmania

Selective toxicity index, which is used as an indicator of selective toxicity against leishmania, was calculated according to the following equation to determine the effect of the compound of the invention.

$$\text{Selective toxicity index} = (EC_{50}\text{ value of sample against rat L6 cells}) / (EC_{50}\text{ value of sample against leishmania protozoa})$$

The $EC_{50}$ values and selective toxicity indexes of the compound of the invention and positive control against leishmania protozoa and rat L6 cells are respectively shown in table 4.

Table 4

| Compound | $EC_{50}$ (μg/ml) | | Selective toxicity |
|---|---|---|---|
| | *Leishmania donovani* | cytotoxicity L6 | |
| A-1 | 0.127 | 37.93 | 299 |
| A-2 | 0.002 | 5.5 | 2800 |
| A-10 | 0.08 | 49 | 6100 |
| A-17 | 0.127 | 8.41 | 66 |
| A-21 | 0.343 | 6.01 | 18 |

(continued)

| Compound | $EC_{50}$ (μg/ml) | | Selective toxicity |
|---|---|---|---|
| | *Leishmania donovani* | cytotoxicity L6 | |
| B-2 | 0.249 | 26.97 | 27 |
| B-12 | 0.167 | 6.9 | 41 |
| B-28 | 0.037 | 5.01 | 135 |
| B-29 | >0.04 | 1.98 | >49 |
| B-30 | 0.11 | 7.46 | 68 |
| B-32 | 0.2195 | 15.88 | 72 |
| B-33 | 0.2015 | 9.83 | 49 |
| B-39 | 0.049 | 3.72 | 76 |
| C-5 | 0.093 | 1.3 | 14 |
| S-3 | 0.53 | >90 | > 170 |
| Miltefosine | 0.11 | - | - |

As clearly shown in the table, the compound of the invention showed proliferation inhibition effect equivalent or exceeding that of miltefosine (anti-leishmania (Chagas' disease) drug currently in clinical use) as a positive control, and that of control compound S - 3 against leishmania protozoa. Based on these findings, it was determined that the compound of the invention is an effective therapeutic agent against leishmania.

14. Test to evaluate therapeutic effect of the compound of the invention in malaria infected mouse (in vivo test)
For a test to evaluate therapeutic effect of the compound, 4-day suppressive test which is commonly used for in vivo test to evaluate activity of anti-malaria compound was used. In this experiment, rodent malaria protozoa (Plasmodium berghei NK 65 strain) were used. The infected blood was collected from 5-weeks age (SPF) ICR strain male mouse which were infected intraperitoneally and successively cultured. From the caudal vein of the infected mouse, blood was collected to calculate infection rate. After confirming that infection rate was adequately elevated (10% to 20% infection), the malaria infected blood was collected from the heart of the mouse. The infection rate and count of the red blood cells (cells/mL) were calculated. The infected red blood cell was diluted with PBS buffer to achieve 1.0 x $10^{-4}$ protozoa per 0.2 mL dose. The infected cells were injected to the caudal vein of uninfected mouse (ICR strain, male, 5-weeks age) to cause infection. Five mice were made into one group, and the test compound was administered in predefined concentration. From 2 hours after infection, the compound was administered every 24 hours for straight 4 days.
The compound of the invention to be used in this test was dissolved in saline (Otsuka Pharmaceutical Co., Ltd.) to obtain test solution of predefined concentration. When the compound was not dissolved in saline, it was dissolved in SSV (saline containing 0.5% sodium=carboxymethyl cellulose, 0.5% benzyl alcohol, 0.4% Tween 80) to prepare test solution. For the control group (non-treatment group), only saline (Otsuka) was administered. Positive controls S - 1 to S - 5 were dissolved in saline (Otsuka) or SSV in predetermined concentration to prepare test solution. When the compound was not dissolved in saline, it was dissolved in SSV. After measuring weight of each mouse, the dose for each mouse was calculated to achieve 5.0 to 40 mg concentration for weight 1kg.
Five mice were made into one group, and the test compound was administered intraperitoneally at predefined concentration. From 2 hours after infection, the compound was administered every 24 hours for straight 4 days. Subsequent to the 24 hours after last treatment, blood was collected from the tail of the mouse to prepare thin layer smear in order to count malaria protozoa infection in the treatment group and the control group under microscope. The mice showing maximum value and minimum value were excluded from the average calculation (average was calculated for three mice), to determine malaria protozoa infection rate (Parasitemia).

Using malaria protozoa infection rate thus calculated, recovery rate obtained from the test compound (suppression) was determined.

$$\text{Recovery rate: suppression (\%)} = (b - a)/b \times 100$$

a: Protozoa infection rate in the treatment group

b: Protozoa infection rate in the control group

$$\text{Mean survival days: MSD (day)} = c - d$$

c: Average days from the initial treatment (date of malaria infection) to the date of death for the treatment group (5 mice)
d: Average days from the date of malaria infection to the date of death for the non-treatment (control) group (5 mice)

Condition of treated mice, such as change in weight and fur, was observed to evaluate adverse effect including acute toxicity of the drug. Tables 5 to 7 illustrate the recovery rate (%) and survival days (day) of malaria infected mice treated with intraperitoneal injection of the test compound and the control compound at predefined concentration. The meaning of symbols in the table is as follows.

* ND: Survival day was not calculated
** All: mice died during treatment period due to acute toxicity of the drug.

Table 5

| Test Compound No. | Dose (mg/kg/d) | Recovery Rate (%) | MSD |
|---|---|---|---|
| A-1 | 10 | 54.1 | ND (*) |
| A-1 | 25 | 62.7 | 2.0 |
| A-2 | 10 | 54.6 | ND (*) |
| A-14 | 10 | 64.5 | ND (*) |
| A-15 | 25 | 60.3 | 2.0 |
| A-18 | 25 | 40.99 | 7.0 |
| A-19 | 25 | 51.1 | 3.0 |
| B-2 | 10 | 79.8 | ND (*) |
| B-2 | 20 | 97.4 | ND (*) |
| B-2 | 25 | 97.1 | 19.3 |
| B-2 | 40 | 99.1 | ND (*) |
| B-11 | 25 | 82.7 | ND (*) |
| B-12 | 25 | 88.4 | ND (*) |
| B-13 | 25 | 95.6 | ND (*) |
| B-16 | 20 | 97.43 | ND (*) |
| B-17 | 25 | 73.1 | 6.8 |
| B-18 | 25 | 22.39 | 7.0 |
| B-20 | 25 | 56.1 | 6.3 |
| B-21 | 25 | 80.4 | 7.0 |
| B-22 | 25 | 72.4 | 9.0 |
| B-23 | 25 | 85.1 | 7.0 |
| B-26 | 25 | 64.3 | 2.0 |
| B-27 | 25 | 55.4 | 2.0 |

Table 6

| Test Compound No. | Dose (mg/kg/d) | Recovery Rate (%) | MSD |
|---|---|---|---|
| B-28 | 25 | 98.3 | ND (*) |
| B-29 | 25 | 95.0 | ND (*) |
| B-30 | 10 | 70.8 | ND (*) |
| B-30 | 20 | 92.4 | ND (*) |
| B-30 | 25 | 96.4 | 16. 0 |
| B-30 | 40 | 97.8 | ND (*) |
| B-31 | 25 | 90.6 | 1.7 |
| B-32 | 25 | 79.5 | 4.8 |
| B-33 | 25 | 70.5 | 8.7 |
| B-35 | 25 | 63.5 | 3.5 |
| B-36 | 25 | 95.5 | 1.7 |
| B-37 | 25 | 94.5 | ND (*) |
| B-38 | 25 | 95.7 | ND (*) |
| B-39 | 25 | 77.7 | 4.5 |
| B-41 | 25 | 58.5 | 1.3 |
| C-2 | 5.0 | 32.1 | ND (*) |
| C-3 | 5.0 | 43.9 | ND (*) |
| C-4 | 5.0 | 37.9 | ND (*) |
| S-1 | 10 | 30.0 | ND (*) |
| S-1 | 25 | toxic | * * |
| S-2 | 10 | 27.0 | ND (*) |
| S-3 | 5.0 | 29.8 | 0 |
| S-4 | 5.0 | 17.1 | ND (*) |

Table 7

| Test Compound No. | Dose (mg/kg/d) | Recovery Rate (%) | MSD |
|---|---|---|---|
| S-4 | 10 | 36.0 | ND (*) |
| S-4 | 25 | toxic | * * |
| S-5 | 5.0 | 41. 0 | ND (*) |
| S-6 | 50 | 25.9 | 0 |
| Quinine | 10 | 18.6 | 1.0 |
| Control | - | 0 | 0 |

As clearly shown in the above tables, the compound of the invention improved recovery rate of malaria and significantly prolonged survival compared to S - 1 to S - 6 compounds as controls. Thus, the compound is an effective therapeutic agent against malaria. Compared to Chloroquine which is currently in clinical use, the compound of the invention shows equivalent or exceeding recovery rate. The control compounds S - 2 and S - 4 had such high toxicity

that it caused death at 25 mg/kg/day dose due to acute toxicity. On the contrary, many compounds of the invention showed no acute toxicity at 25 mg/kg/day dose. Therefore, the compound of the invention is an effective therapeutic agent against malaria with improved adverse effect profile.

Synthesis of compound of the invention

Synthesis of compound A - 14 3-ethyl-5-(1-methyl-1H-quinoline-2-ylidene)-2-methylsulfani 1-4-oxo-4,5-dihydro-3-thiazolium=p-toluenesulfonate 244 mg and 2-amino-1-methylpyridinium=p-toluenesulfonate 140 mg were dissolved into 2.5mL acetonitrile. To the solution, 0.21 mL triethylamine was agitatedly titrated at 70°C. The agitation was continued for 6 hours at same temperature. To the mixture, 2. 5 mL ethyl acetate was added at 70°C, and the mixture was cooled to ambient temperature and left for 30 minutes. The crystal was sucked, filtered, and washed with acetonitrile-ethyl acetate mixture (1:1 v/v). The resultant coarse crystal was dissolved into chloroform-methanol mixture solution (1: 1 v/v), then introduced into a column filled with strong base ion-exchange resin (Amberite IRA-400, Sigma-Aldrich Japan K.K.) to elute with chloroform-methanol mixture solution (1:1 v/v). The elusion was concentrated under reduced pressure to obtain solid substance. The solid was recrystallized using methanol-ethyl acetate mixture to obtain the intended compound. yield 147 mg (71%), melting point 239 - 240°C

Synthesis of compound B - 2

1) Synthesis of 3-ethyl-5-(1-methyl)-1H-piridine-2-ylidene)-2-thioxothiazol idine-4-on

A mixture of methylsulfanylpyridinium=p-toluensulfonate 7.79g and 3-ethyl-2thioxothiazolidine-4-on 4.03 g was dissolved in 25 mL acetonitrile and cooled to 0°C. To the solution, triethylamine 10.5 mL was agitatedly titrated at 0°C. The reaction temperature was raised to 10°C, and agitation was continued for 4 hours. The resultant crystal was sucked, filtered, washed with acetonitrile, and dried under reduced pressure to obtain intended compound.
yield 5.37g (85% yield), melting point 148 - 150 °C

2) Synthesis of 3-ethyl-5-(1-methyl-1H-pyridine-2-ylidene)-2-methylsulfanyl -4-oxo-4,5-dihydro-3-thiazolium=p-toluenesulfonate

The mixture of 3-ethyl-5-(1-methyl-1H-piridine-2-ylidene)-2-thioxothiazoli dine-4-on 5.06 g and p-toluenesulfonate methyl 11. 2 g was heated to 130°C and agitated for 4 hours. The mixture was cooled to ambient temperature, and added with 20 mL ethyl acetate and agitated for 30 minutes. The resultant crystal was sucked, filtered, washed with ethyl acetate, and dried under reduced pressure at ambient temperature to obtain intended compound. yield 8.45 g (96%), melting point 156 to 158 °C

3) Synthesis of 6-chloro-2-(3-ethyl-4-oxothiazolidine-2-ylideneamino)-3-met hyl-3-benzothiazolium=bromide

The mixture of 2-amino-6-chloro-3-methyl-3-benzothiazolium=p-toluensulfona te 1.74 g and ethylisothyocianate 0.42 mL was dissolved in 2.5 mL pyridine. To the solution, 0.66 mL triethylamine was titrated at ambient temperature, then the resultant solution was heated to 110°C, and agitated for 45 minutes. Ice water was added to the mixture. The resultant precipitate was sucked, filtered and washed with distilled water, then dried under reduced pressure. To the resultant coarse product, bromoacetic acid 1.29 g and acetic acid 4 mL were added, and the mixture was agitated at 90°C for 30 minutes. After cooling to the ambient temperature, the mixture was added with 10 mL diethyl ether and left for 30 minutes. The resultant crystal was sucked, filtered, washed with diethyl ether, and dried under reduced pressure. The resultant coarse crystal was recrystallized using methanol to obtain the intended compound. yield 1.72g (90%), melting point 233 - 235°C

4) Synthesis of compound B-2 6-chloro-2-(3-ethyl-4-oxothiazolidine-2-ylideneamino)-3-met hyl-3-benzothiazolium=bromide 203 mg and

3-ethyl-5-(1-methyl-1H-pyridine-2-ylidene)-2-methylsulfanyl -4-oxo-4,5-dihydro-3-thiazolium=p-toluenesulfonate 182 mg was dissolved in acetonitrile 5 mL. To the solution, triethylamine 0.21 mL was titrated at ambient temperature, then the resultant solution was agitated at 70°C for 15 hours. The mixture was cooled to ambient temperature then the resultant precipitate was sucked, filtered, and washed with acetonitrile. The resultant coarse crystal was dissolved in chloroform-methanol mixed solution (1:1 v/v), and introduced through a column filled with strong base ion exchange resin (Amberlite IRA-400 by Aldrich), then eluted with chloroform-methanol mixture (1:1 v/v). The elution was concentrated under reduced pressure to obtain solid. The solid was recrystallized using methanol-ethyl acetate mixture to obtain the

intended compound.
yield 186 mg (77%), melting point 203 - 205°C (decomp.)

1 Synthesis of compound C-4

1) Synthesis of 2-amino-4-(4-chlorophenyl)-3-methyl-3-thiazolium=p-toluenes ulfonate

2-amino-4-(4-chlorophenyl) thiazole 500 mg and p-toluenesulfonate methyl 0.39mL was dissolved in acetonitrile 0.59 mL, and agitated at 80°C for 20 hours. The mixture was cooled to ambient temperature. The resultant precipitate was sucked, filtered and washed with ethyl acetate, then dried under reduced pressure to obtain the intended compound.
yield 704mg (75%)

2) Synthesis of compound C-4

2-amino-4-(4-chlorophenyl)-3-methyl-3-thiazolium=p-toluenes ulfonate 76 mg and 3-ethyl-5-(2-(3-methyl-3H-benzothiazole-2-ylidene)ethyliden e)-2-methylsulfanyl-4-oxo-4,5-dihydro-3-thiazolium=p-tluene sulfonate 100 mg was dissolved in acetonitrile 6.4 mL. To the solution, triethylamine was titrated at ambient temperature 0.077 mL, then the resultant solution was agitated at 70°C for 5.5 hours. The mixture was cooled to ambient temperature, and the resultant precipitate was sucked, filtered and washed with acetonitrile. The obtained coarse crystal was dissolved in chloroform-methanol mixture (1:1 v/v), then introduced through a column filled with strong base ionexchange resin (Amberlite IRA-400 by Aldrich). The resultant product was eluted by chloroform-methanol mixture (1:1 v/v). The elution was concentrated under reduced pressure to obtain solid. The solid was recrystallized by methanol-ethyl acetate mixture to obtain the intended compound.
yield 47 mg (44%)

Other compounds of the invention were synthesized using methods similar to the above. The spectrum data of each compound of the present invention is as follows:
A - 1 Mp 263-265 °C(decomp.); IR (KBr) 1647, 1535, 1489, 1416, 1352 $cm^{-1}$; $^1H$ NMR (DMSO-$d_6$) δ 8.37 (1H, d, *J*= 2.2 Hz), 8.05 (1H, d, *J*= 8.3 Hz), 8.02 (1H, d, *J*= 9.0 Hz), 7.86-7.81 (2H, m), 7.59 (1H, dd, *J*= 7.3, 8.3 Hz), 7.42 (1H, dd, *J*= 7.6, 7.6 Hz), 4.27-4.02 (5H, m), 4.02 (3H, s), 1.35 (3H, t, *J*= 7.1 Hz); MS (FAB) *m*/*z* 473 ($M^+$).

A - 2 mp 291-293 °C(decomp.); IR (KBr) 1653, 1609, 1531, 1479, 1394, 1167 $cm^{-1}$; $^1H$ NMR (DMSO-$d_6$) δ 8.38 (1H, s), 8.34 (1H, d, *J*= 9.0 Hz), 8.22 (1H, d, *J*= 7.3 Hz), 8.08 (1H, d, *J*= 8.8 Hz), 8.01 (1H, d, *J*= 8.1 Hz), 7.98 (1H, d, *J*= 9.0 Hz), 7.92 (1H, dd, *J*= 7.6, 8.1 Hz), 7.81 (1H, d, *J* = 8.8 Hz), 7.65 (1H, dd, *J*= 7.3, 7.6 Hz), 4.22-4.13 (5H, m), 4.01 (3H, s), 1.35 (3H, t, *J*= 7.1 Hz); MS ($FAB^+$) *m*/*z* 467 ($M^+$).

A - 4 mp 232-234 °C(decomp.); IR (KBr) 1641, 1524, 1497, 1445, 1375, 1281 $cm^{-1}$; $^1H$ NMR (DMSO-$d_6$) δ 8.49 (1H, d, *J*= 6.6 Hz), 8.42 (1H, d, *J*= 8.3 Hz), 8.33 (1H, s), 8.04 (1H, dd, *J*= 7.1, 8.3 Hz), 7.92 (1H, d, *J*= 8.8 Hz), 7.77 (1H, d, *J*= 8.8 Hz), 7.34 (1H, dd, *J*= 6.6, 7.1 Hz), 4.26 (3H, s), 4.17 (2H, q, *J*= 7.1 Hz), 3.96 (3H, s), 1.32 (3H, t, *J*= 7.1 Hz); MS (FAB) m/z 417 ($M^+$).

A-1 4 mp 239-240°C (decomp.); IR (KBr) 1612, 1508, 1481, 1394, 1165 $cm^{-1}$; $^1H$ NMR (DMSO-$d_6$) δ 8.74 (1H, d, *J*= 6.3 Hz), 8.33 (1H, dd, *J*= 8.1, 8.1 Hz), 7.98 (1H, d, *J*= 9.3 Hz), 7.90 (1H, d, *J* = 8.5 Hz), 7.86-7.72 (4H, m), 7.47 (2H, dd, *J* = 6.3, 8.1 Hz), 4.04 (3H, s), 4.01 (2H, q, *J* = 7.1 Hz), 3.94 (3H, s), 1.29 (3H, t, *J*= 7.1 Hz); MS ($FAB^+$) m/z 377 ($M^+$).

A- 1 5 mp 262-264 °C(decomp.); IR (KBr) 1614, 1541, 1487, 1435, 1367, 1198 $cm^{-1}$; $^1H$ NMR (DMSO-$d_6$) δ 8.85 (1H, d, *J*= 7.3 Hz), 8.68 (1H, d, *J*= 9.0 Hz), 8.49 (1H, d, *J*= 8.8 Hz), 8.31 (1H, s), 8.22 (1H, d, *J*= 6.6 Hz), 7.88 (1H, d, *J*= 9.0 Hz), 7.81 (1H, dd, *J*= 6.8, 8.8 Hz), 7.59 (1H, d, *J*= 7.3 Hz), 7.05 (1H, dd, *J*= 6.6, 6.8 Hz), 4.27 (3H, s), 4.19-4.06 (5H, m), 1.32 (3H, t, *J*= 7.1 Hz); MS (FAB) m/z 411 ($M^+$).

A- 1 6 mp 267-269 °C (decomp.); IR (KBr) 1649, 1558, 1493, 1418, 1202 $cm^{-1}$; $^1H$ NMR (DMSO-$d_6$) δ 9.08 (1H, d, *J*= 6.8 Hz), 8.59 (1H, d, *J*= 9.0 Hz), 8.46 (1H, s), 7.96 (1H, d, *J*= 9.0 Hz), 7.92 (1H, d, *J* = 7.8 Hz), 7.76 (1H, d, *J* = 6.8 Hz), 7.62 (1H, d, *J* = 8.3 Hz), 7.51 (1H, dd, *J*= 7.3, 7.8 Hz), 7.33 (1H, dd, *J*= 7.3, 8.3 Hz), 4.39 (3H, s), 4.15 (2H, q, *J*= 7.1 Hz), 3.95 (3H, s), 1.36 (3H, t, *J*= 7.1 Hz); MS (FAB) *m*/*z* 467 ($M^+$).

A - 1 7 mp >250 °C (decomp.); IR (KBr) 1636, 1539, 1489, 1407, 1350 $cm^{-1}$; $^1H$ NMR (DMSO-$d_6$) δ 8.36 (1H, d, *J*

*=* 2.2 Hz), 8.09-8.00 (3H, m), 7.84 (1H, dd, *J* = 2.2, 9.0 Hz), 7.49 (1H, dd, *J*= 1.7, 8.3 Hz), 4.22 (2H, q, *J*= 7.1 Hz), 4.18 (3H, s), 4.03 (3H, s), 1.35 (3H, t, *J*= 7.1 Hz); MS (FAB) *m*/*z* 507 ($M^+$).

A - 1 9 $^1$H NMR (DMSO-$d_6$) δ8.64 (1H, d, *J*= 6.3 Hz), 8.24 (1H, t, *J* = 7.6 Hz), 8.05 (1H, d, *J* = 6.5 Hz), 7.92 (1H, d, *J* = 6.3 Hz), 7.90 (1H, d, *J*= 7.2 Hz), 7.34 (1H, dd, *J*= 6.5, 7.2 Hz), 7.09 (1H, dd, *J*= 7.6, 7.2 Hz), 4.11 (3H, s), 4.03 (2H, q, *J*= 7.0 Hz), 4.00 (3H, s), 1.23 (3H, t, *J*= 7.0 Hz); MS (FAB) *m*/*z* 368 ($M^+$).

B-2 mp 203-205 °C (decomp.); IR (KBr) 1636, 1541, 1489, 1373, 1271, 1005 $cm^{-1}$ ; $^1$H NMR (DMSO-$d_6$) δ8.44 (1H, d, *J*= 8.78 Hz), 8.37 (1H, d, *J*= 2.20 Hz), 8.29 (1H, d, *J*= 6.59 Hz), 7.98 (1H, d, *J*= 8.78 Hz), 7.85 (1H, dd, *J*= 6.83, 8.78 Hz), 7.80 (1H, dd, *J* = 2.20, 8.78 Hz), 7.10 (1H, dd, *J*= 6.59, 6.83 Hz), 4.26-4.15 (7H, m), 4.00 (3H, s), 1.46-1.25 (6H, m); MS (FAB) *m*/*z* 544 ($M^+$).

B-3 mp >250 °C (decomp.); IR (KBr) 1636, 1578, 1489, 1435, 1366, 1155 $cm^{-1}$; $^1$H NMR (DMSO-$d_6$) δ 9.06 (1H, d, *J*= 2.4 Hz), 8.46-8.46 (2H, m), 8.15 (1H, d, *J*= 6.1 Hz), 7.89 (1H, d, *J*= 9.3 Hz), 7.73 (1H, dd, *J*= 6.8, 8.5 Hz), 6.97 (1H, dd, *J*= 6.1, 6.8 Hz), 4.11 (3H, s), 4.09-3.97 (7H, m), 1.29-1.20 (6H, m); MS (FAB) *m*/*z* 488 ($M^+$).

B-4 mp >300 °C (decomp.); IR (KBr) 1653, 1616, 1483, 1435, 1396, 173 $cm^{-1}$; $^1$H NMR (DMSO-$d_6$) δ 8.81 (1H, d, *J*= 6.3 Hz), 8.39 (1H, dd, *J*= 7.2, 8.7 Hz), 7.98-7.85 (2H, m), 7.84-7.70 (4H, m), 7.54 (1H, dd, *J*= 6.3, 7.2 Hz), 7.44 (1H, dd, *J*= 7.2, 7.5 Hz), 4.06 (3H, s), 4.04-3.97 (4H, m), 3.95 (3H, s), 1.28 (3H, t, *J*= 7.0 Hz), 1.24 (3H, t, *J*= 7.0 Hz); MS (FAB) *m*/*z* 504 ($M^+$).

B-5 mp >300 °C (decomp.); IR (KBr) 1624, 1541, 1489, 1437, 1375, 1155 $cm^{-1}$; $^1$H NMR (DMSO-$d_6$) δ 8.66 (1H, d, *J*= 6.6 Hz), 8.45 (1H, d, *J*= 8.5 Hz), 8.34 (1H, dd, *J*= 7.3, 8.5 Hz), 8.12 (1H, d, *J* = 7.3 Hz), 7.89 (1H, d, *J* = 8.3 Hz), 7.70 (1H, dd, *J*= 7.3, 7.3 Hz), 7.60-7.45 (6H, m), 6.94 (1H, dd, *J*= 6.6, 6.6 Hz), 4.08 (5H, m), 3.76 (3H, s), 1.27 (3H, t, *J*= 7.1 Hz); MS (FAB) *m*/*z* 502 ($M^+$).

B-6 mp 258-260 °C (decomp.); IR (KBr) 1624, 1541, 1491, 1437, 1373, 1155 $cm^{-1}$ ; $^1$H NMR (DMSO-$d_6$) δ 8.73 (1H, d, *J*= 6.3 Hz), 8.44 (1H, d, *J*= 8.3 Hz), 8.35 (1H, dd, *J*= 7.1, 7.1 Hz), 8.14 (1H, d, *J*= 6.6 Hz), 7.88 (1H, d, *J*= 7.8 Hz), 7.71 (1H, dd, *J*= 7.1, 7.1 Hz), 7.48 (1H, dd, *J*= 6.3, 7.8 Hz), 6.95 (1H, dd, *J*= 6.6, 8.3 Hz), 4.11 (3H, s), 4.08-3.97 (7H, m), 1.27 (3H, t, *J*= 7.1 Hz), 1.23 (3H, t, *J*= 7.1 Hz); MS (FAB) *m*/*z* 454 ($M^+$).

B - 8 mp >250 °C (decomp.); IR (KBr) 1636, 1541, 1487, 1435, 1373, 1153 $cm^{-1}$; $^1$H NMR (DMSO-$d_6$) δ 8.45 (1H, d, *J* = 8.54 Hz), 8.26 (1H, d, *J*= 6.59 Hz), 7.83 (1H, dd, *J* = 7.32, 8.54 Hz), 7.77 (1H, s), 7.71-7.64 (4H, m), 7.09 (1H, dd, *J*= 6.59, 7.32 Hz), 4.22 (2H, q, *J* = 7.07 Hz), 4.16 (3H, s), 4.13 (2H, q, *J*= 7.32 Hz), 3.71 (3H, s), 1.38-1.28 (6H, m); MS (FAB) *m*/*z* 570 ($M^+$);

B - 1 1 mp >300 °C (decomp.); IR (KBr) 1636, 1541, 1487, 1437, 1373, 1161 $cm^{-1}$; $^1$H NMR (DMSO-$d_6$) δ 8.45 (1H, d, *J*= 8.8 Hz), 8.37 (1H, d, *J*= 2.2 Hz), 8.29 (1H, d, *J*= 6.6 Hz), 7.92-7.83 (2H, m), 7.78 (1H, dd, *J*= 2.2, 8.8 Hz), 7.66-7.52 (5H, m), 7.12 (1H, dd, *J*= 6.6, 7.1 Hz), 4.30 (2H, q, *J*= 7.1 Hz), 4.16 (3H, s), 3.61 (3H, s), 1.42 (3H, t, *J*= 7.1 Hz); MS (FAB) *m*/*z* 592 ($M^+$).

B - 1 2 mp >300 °C (decomp.); IR (KBr) 1661, 1537, 1489, 1385, 1001 $cm^{-1}$; $^1$H NMR (DMSO-$d_6$) δ. 8.39 (1H, d, *J* = 2.2 Hz), 8.03 (1H, d, *J* = 8.8 Hz), 7.83 (1H, dd, *J* = 2.2, 8.8 Hz), 4.21-4.12 (4H, m), 4.02 (3H, s), 3.96 (2H, t, *J*= 7.6 Hz), 3.48 (3H, s), 3.27 (2H, t, *J*= 7.6 Hz), 1.32 (3H, t, *J*= 7.3 Hz); MS (FAB) *m*/*z* 552 ($M^+$).

B - 14 mp >300 °C (decomp.); IR (KBr) 1653, 1609, 1543, 1475, 1389, 1161 $cm^{-1}$; $^1$H NMR (DMSO-$d_6$) δ 8.35 (1H, d, *J*= 2.2 Hz), 8.06 (1H, d, *J*= 9.5 Hz), 7.92-7.87 (2H, m), 7.86-7.76 (3H, m), 7.69-7.58 (6H, m), 7.50 (1H, dd, *J*= 7.0, 7.3 Hz), 4.28 (2H, q, *J*= 7.3 Hz), 4.03 (3H, s), 3.63 (3H, s), 1.43 (3H, t, *J*= 7.3 Hz); MS (FAB) *m*/*z* 642 ($M^+$).

B - 15 mp > 300 °C (decomp.); IR (KBr) 1653, 1609, 1541, 1477, 1394 $cm^{-1}$; $^1$H NMR (DMSO-$d_6$) δ 8.34 (1H, d, *J*= 2.2 Hz), 8.07 (1H, d, *J*= 9.8 Hz), 8.00 (1H, d, *J*= 9.0 Hz), 7.93-7.76 (5H, m), 7.49 (1H, dd, *J*= 7.3, 7.6 Hz), 4.27-4.15 (4H, m), 4.04 (3H, s), 4.02 (3H, s), 1.43-1.31 (6H, m); MS (FAB) *m*/*z* 594 ($M^+$).

B - 1 6 mp 207-208 °C (decomp.); IR (KBr) 1636, 1528, 1487, 1437, 1373, 1155 $cm^{-1}$; $^1$H NMR (DMSO-$d_6$) δ 8.45 (1H, d, *J*= 8.8 Hz), 8.36 (1H, d, *J*= 2.2 Hz), 8.31 (1H, d, *J*= 6.8 Hz), 7.97 (1H, d, *J*= 9.0 Hz), 7.86 (1H, dd, *J*= 6.8, 8.8 Hz), 7.79 (1H, d, *J*= 9.0 Hz), 7.49 (2H, d, *J*= 7.1 Hz), 7.41-7.28 (3H, m), 7.13 (1H, dd, *J*= 6.8, 6.8 Hz), 5.34 (2H, s), 4.25 (2H, q, *J*= 7.3 Hz), 4.18 (3H, s), 3.96 (3H, s), 1.36 (3H, t, *J*= 7.3 Hz); MS (FAB) m/z 606 ($M^+$);

B - 1 7 mp >300 °C (decomp.); IR (KBr) 1638, 1541, 1485, 1439, 1373, 1165 $cm^{-3}$; $^1H$ NMR (DMSO-$d_6$) δ 8.46 (1H, d, *J*= 8.9 Hz), 8.37 (1H, d, *J*= 1.5 Hz), 8.28 (1H, d, *J*= 6.5 Hz), 7.91-7.82 (3H, m), 7.78 (1H, dd, *J*= 1.5, 8.9 Hz), 7.49 (2H, d, *J*= 8.9 Hz), 7.16 (2H, d, *J*= 8.7 Hz), 7.11 (1H, t, *J*= 6.5 Hz), 4.30 (2H, q, *J*= 7.2 Hz), 4.16 (3H, s), 3.86 (3H, s), 3.64 (3H, s), 1.41 (3H, dd, *J*= 6.5, 7.2 Hz); MS (FAB) *m*/*z* 622 ($M^+$).

B - 1 9 mp 252-253 °C (decomp.); IR (KBr) 1645, 1545, 1489, 1439, 1375, 1163 $cm^{-1}$; $^1H$ NMR (DMSO-$d_6$) δ 8.44 (1H, d, *J*= 8.8 Hz), 8.25 (1H, d, *J*= 6.3 Hz), 8.00 (1 H, d, *J*= 4.4 Hz), 7.82 (1H, dd, *J*= 7.3, 8.8 Hz), 7.75 (1H, d, *J*= 4.4 Hz), 7.07 (1H, dd, *J*= 6.3, 7.3 Hz), 6.01-5.89 (1H, m), 5.32-5.21 (2H, m), 4.67 (2H, d, *J*= 5.4 Hz), 4.20 (2H, q, *J*= 7.3 Hz), 4.15 (3H, s), 3.86 (3H, s), 1.33 (3H, t, *J*= 7.3 Hz); MS (FAB) *m*/*z* 472 ($M^+$).

B-2 0 $^1H$ NMR (DMSO-$d_6$) 88.13 (1H, d, *J* = 6.7 Hz), 8.04 (1H, dd, *J* = 4.2, 7.6 Hz), 7.77 (1H, d, *J* = 4.2 Hz), 7.20 (1H, dd, *J* = 6.7, 7.6 Hz), 4.14-4.08 (7H, n, *J* = 6.5, 7.2 Hz), 3.91 (3H, s), 1.31 (6H, m); MS (FAB) *m*/*z* 501 ($M^+$).

B - 21 mp 233-234 °C (decomp.); IR (KBr) 1638, 1541, 1489, 1437, 1375, 1157 $cm^{-1}$; $^1H$ NMR (DMSO-$d_6$) δ 8.46 (1 H, d, *J* = 8.9 Hz), 8.27 (1H, d, *J* = 6.5 Hz), 8.01 (1 H, d, *J* = 4.4 Hz), 7.83 (1H, dd, *J*= 7.2, 8.9 Hz), 7.75 (1H, d, *J* = 4.4 Hz), 7.46 (2H, d, *J*= 7.2 Hz), 7.41-7.28 (3H, m), 7.09 (1H, dd, *J*= 6.5, 7.2 Hz), 5.26 (2H, s), 4.20 (2H, q, *J*= 7.0 Hz), 4.16 (3H, s), 3.85 (3H, s), 1.33 (3H, t, *J*= 7.0 Hz),; MS (FAB) *m*/*z* 522 ($M^+$).

B-22 $^1H$ NMR (DMSO-$d_6$) δ 8.12 (1H, d, *J*= 6.2 Hz), 7.99 (1H, d, *J*= 4.2, Hz), 7.75 (1H, dd, *J* = 4.2, 2.1 Hz), 7.47 (2H, d, *J* = 7.6 Hz), 7.36 (2H, dt, *J* = 1.4, 7.6 Hz), 7.31 (1 H, t, *J* = 7.3 Hz), 7.19 (1H, t, *J*= 6.2 Hz), 5.24 (s, 2H), 4.12 (2H, q, *J*= 6.8 Hz), 4.06 (3H, s), 3.82 (s, 3H), 1.31 (3H, t, *J*= 6.8 Hz); MS (FAB) *m*/*z* 563 ($M^+$).

B - 2 3 mp 200-202 °C (decomp.); $^1H$ NMR (DMSO-$d_6$) δ8.04 (1H, d, *J*= 4.4 Hz), 7.81 (1H, d, *J*= 4.4 Hz), 7.45 (2H, d, *J*= 7.1 Hz), 7.39-7.28 (3H, m), 5.23 (2H, s), 4.13 (2H, q, *J*= 7.1 Hz), 3.94 (2H, t, *J*= 7.6 Hz), 3.86 (3H, s), 3.47 (3H, s), 3.25 (2H, t, *J*= 7.6 Hz), 1.29 (3H, t, *J*= 7.1 Hz); MS (FAB) *m*/*z* 530 ($M^+$).

B - 24 mp >280 °C (decomp.); $^1H$ NMR (DMSO-$d_6$) δ 8.00 (1H, d, *J*= 4.4 Hz), 7.82 (1H, d, *J* = 4.4 Hz), 7.62-7.51 (5H, m), 4.17 (2H, q, *J* = 7.3 Hz), 3.92 (2H, t, *J* = 7.8 Hz), 3.52 (3H, s), 3.43 (3H, s), 3.25 (2H, t, *J*= 7.8 Hz), 1.34 (3H, t, *J*= 7.3 Hz); MS (FAB) *m*/*z* 516 ($M^+$).

B - 2 5 mp >250 °C (decomp.); IR (KBr) 1655, 1545, 1497, 1389, 1167 $cm^{-1}$; $^1H$ NMR (DMSO-$d_6$) δ 8.00 (1H, d, *J* = 4.4 Hz), 7.82 (1H, d, *J*= 4.4 Hz), 7.44 (2H, d, *J* = 8.8 Hz), 7.11 (2H, d, *J*= 8.8 Hz), 4.16 (2H, q, *J*= 7.1 Hz), 3.92 (2H, t, *J*= 7.6 Hz), 3.84 (3H, s), 3.55 (3H, s), 3.42 (3H, s), 3.24 (2H, t, *J*= 7.6 Hz), 1.33 (3H, t, *J*= 7.1 Hz); MS (FAB) *m*/*z* 546 ($M^+$).

B-2 6 mp >300 °C (decomp.); IR (KBr) 1653, 1541, 1491, 1389, 1167 $cm^{-1}$ ; $^1H$ NMR (DMSO-$d_6$) δ 8.39 (1H, d, *J*= 2.2 Hz), 7.91 (1H, d, *J*= 8.8 Hz), 7.79 (1H, dd, *J*= 2.2, 8.8 Hz), 7.48 (2H, d, *J*= 8.8 Hz), 7.15 (2H, d, *J*= 8.8 Hz), 4.21 (2H, q, *J*= 7.3 Hz), 3.94 (2H, t, *J*= 7.6 Hz), 3.85 (3H, s), 3.66 (3H, s), 3.45 (3H, s), 3.26 (2H, t, *J*= 7.6 Hz), 1.37 (3H, t, *J*= 7.3 Hz); MS (FAB) *m*/*z* 630 ($M^+$).

B-2 7 mp 215-217 °C (decomp.); IR (KBr) 1653, 1541, 1504, 1389, 1194 $cm^{-1}$; $^1H$ NMR (DMSO-$d_6$) δ8.07 (1H, d, *J* = 4.4 Hz), 7.82 (1H, d, *J* = 4.4 Hz), 4.14-4.04 (4H, m), 3.93 (2H, t, *J* = 7.6 Hz), 3.89 (3H, s), 3.46 (3H, s), 3.25 (2H, t, *J*= 7.6 Hz), 1.28 (6H, t, *J*= 7.3 Hz); MS (FAB) *m*/*z* 468 ($M^+$).

B-2 8 mp >300 °C (decomp.); IR (KBr) 1636, 1481, 1435, 1373, 1232, 1151 $cm^{-1}$; $^1H$ NMR (DMSO-$d_6$) δ 8.45 (1H, d, *J*= 8.7 Hz), 8.27 (1H, d, *J*= 6.8 Hz), 7.91 (1H, d, *J*= 9.2 Hz), 7.89 (1H, d, *J*= 2.7 Hz), 7.84 (1H, dd, *J*= 6.8, 8.7 Hz), 7.35 (1H, dd, *J*= 2.7, 9.2 Hz), 7.09 (1H, dd, *J*= 6.8, 6.8 Hz), 4.29-4.12 (7H, m), 4.01 (3H, s), 3.87 (3H, s), 1.40-1.30 (6H, m); MS (FAB) *m*/*z* 540 ($M^+$).

B - 2 9 mp 202-204 °C (decomp.); $^1H$ NMR (DMSO-$d_6$) δ 7.95 (1 H, d, *J* = 9.0 Hz), 7.89 (1H, d, *J*= 2.7 Hz), 7.36 (1H, dd, *J*= 2.7, 9.0 Hz), 4.13 (4H, q, *J*= 7.1 Hz), 4.02 (3H, s), 3.92 (2H, t, *J*= 7.8 Hz), 3.87 (3H, s), 3.46 (3H, s), 3.25 (2H, *t, J* = 7.8 Hz), 1.32 (6H, t, *J*= 7.1 Hz); MS (FAB) *m*/*z* 548 ($M^+$).

B - 3 0 mp >300 °C (decomp.); IR (KBr) 1636, 1541, 1489, 1367, 1163, 1007 $cm^{-1}$; $^1H$ NMR (DMSO-$d_6$) δ 8.45 (1H, d, *J*= 8.7 Hz), 8.28-8.23 (2H, m), 7.97 (1H, d, *J*= 8.3 Hz), 7.84 (1 H, dd, *J*= 7.3, 8.7 Hz), 7.75 (1H, dd, *J*= 6.9, 8.3 Hz), 7:58 (1H, dd, *J*= 6.9, 7.3 Hz), 7.08 (1H, t, *J*= 6.4, 7.3 Hz), 4.28-4.14 (7H, m), 4.03 (3H,s), 1.42-1.30 (6H, m); MS (FAB) *m*/*z* 510 ($M^+$).

B - 3 1 mp 235-236 °C (decomp.); IR (KBr) 1653, 1543, 1489, 1364, 1192 $cm^{-1}$; $^1H$ NMR (DMSO-$d_6$) δ 8.29 (1H, d, *J*= 8.3 Hz), 8.02 (1H, d, *J*= 8.7 Hz), 7.77 (1H, dd, *J* = 7.8, 8.3 Hz), 7.62 (1H, dd, *J*= 7.8, 8.3 Hz), 4.19-4.10 (4H, m), 4.05 (3H, s), 3.90 (2H, t, *J*= 7.3 Hz), 3.46 (3H, s), 3.23 (2H, t, *J*= 7.3 Hz), 1.36-1.29 (6H, m); MS (FAB) *m*/*z* 518 ($M^+$).

B - 3 2 mp >300 °C (decomp.); IR (KBr) 1636, 1541, 1477, 1437, 1371, 1153 $cm^{-1}$; $^1H$ NMR (DMSO-$d_6$) δ 8.44 (1H, d, *J*= 9.0 Hz), 8.28 (1H, d, *J*= 6.3 Hz), 8.17 (1H, dd, *J* = 2.9, 8.1 Hz), 8.01 (1H, dd, *J*= 9.0, 9.3 Hz), 7.85 (1H, dd, *J*= 6.8, 9.0 Hz), 7.66 (1H, dd, *J*= 2.7, 9.0 Hz), 7.10 (1H, dd, *J*= 6.3, 6.8 Hz), 4.30-4.12 (7H, m), 4.01 (3H, s), 1.41-1.30 (6H, m); MS (FAB) *m*/*z* 528 ($M^+$).

B - 3 3 mp >250 °C (decomp.); IR (KBr) 1655, 1541, 1489, 1362, 1209, 1001 $cm^{-1}$; $^1H$ NMR (DMSO-$d_6$) δ8.20 (1H, d, *J*= 2.9, 8.1 Hz), 8.06 (1H, dd, *J* = 9.0, 9.3 Hz), 7.69 (1H, dd, *J* = 2.9, 9.0 Hz), 4.20-4.10 (4H, m), 4.03 (3H, s), 3.95 (2H, *t*, *J*= 7.6 Hz), 3.48 (3H, s), 3.27 (2H, t, *J*= 7.6 Hz); 1.32 (6H, t, *J*= 7.1 Hz); MS (FAB) *m*/*z* 536 ($M^+$).

B - 3 4 mp 299-300 °C (decomp.); IR (KBr) 1645, 1541, 1489, 1437, 1377, 1163 $cm^{-1}$; $^1H$ NMR (DMSO-$d_6$) δ 8.45 (1H, d, *J*= 8.7 Hz), 8.26 (1H, d, *J*= 6.9 Hz), 8.03 (1H, d, *J*= 4.6 Hz), 7.82 (1H, dd, *J*= 7.3, 8.7 Hz), 7.75 (1H, d, *J*= 4.6 Hz), 7.07 (1H, dd, *J*= 6.9, 7.3 Hz), 5.16-5.09 (1H, m), 4.20 (2H, q, *J*= 6.9 Hz), 4.16 (3H, s), 3.89 (3H, s), 1.58 (6H, d, *J*= 6.9 Hz), 1.33 (3H, t, *J*= 6.9 Hz); MS (FAB) m/z 474 ($M^+$).

B - 35 mp >250 °C (decomp.); IR (KBr) 1655, 1541, 1500, 1391, 1170 $cm^{-1}$; $^1H$ NMR (DMSO-$d_6$) δ 8.13 (1H, d, *J*= 4.6 Hz), 7.86 (1H, d, *J*= 4.6 Hz), 5.13-5.05 (1H, m), 4.08 (2H, q, *J*= 7.3 Hz), 4.00-3.90 (5H, m), 3.47 (3H, s), 3.25 (3H, s), 1.57 (6H, d, *J*= 6.9 Hz), 1.29 (3H, t, *J*= 7.3 Hz); MS (FAB) *m*/*z* 482 ($M^+$).

B - 3 6 mp > 300 °C; $^1H$ NMR (DMSO-$d_6$) δ8.45 (1H, d, *J*= 8.8 Hz), 8.38 (1H, d, *J*= 2.2 Hz), 8.30 (1H, d, *J*= 6.3 Hz), 7.99 (1H, d, *J*= 8.8 Hz), 7.86 (1H, dd, *J*= 7.1, 8.8 Hz), 7.81 (1H, dd, *J*= 2.2, 8.8 Hz), 7.11 (1H, dd, *J*= 6.3, 7.1 Hz), 5.26-5.17 (1H, m), 4.23 (2H, q, *J*= 7.1 Hz), 4.18 (3H, s), 4.00 (3H, s), 1.61 (6H, d, *J*= 6.8 Hz), 1.36 (3H, t, *J*= 7.1 Hz); MS (FAB) m/z 558 ($M^+$).

B - 37 mp >300 °C (decomp.); IR (KBr) 1649, 1531, 1493, 1410, 1381, 1169 $cm^{-1}$; $^1H$ NMR (DMSO-$d_6$) δ8.40 (1H, d, *J* = 2.2 Hz), 8.03 (1H, d, *J* = 9.0 Hz), 7.83 (1H, dd, *J* = 2.2, 9.0 Hz), 5.22-5.15 (1H, m), 4.16 (2H, q, *J*= 7.3 Hz), 4.02 (3H, s), 3.96 (2H, t, *J*= 7.8 Hz), 3.49 (3H, s), 3.27 (2H, t, *J*= 7.8 Hz), 1.59 (6H, d, *J*= 6.8 Hz), 1.32 (3H, t, *J*= 7.3 Hz); MS (FAB) *m*/*z* 566 ($M^+$).

B - 38 mp 174-176 °C; $^1H$ NMR (DMSO-$d_6$) δ8.44 (1H, d, *J* = 8.8 Hz), 8.37 (1 H, d, *J* = 2.0 Hz), 8.29 (1H, d, *J*= 6.3 Hz), 7.97 (1H, d, *J*= 8.8 Hz), 7.87 (1H, dd, *J*= 7.3, 8.8 Hz), 7.79 (1H, dd, *J* = 2.0, 8.8 Hz), 7.10 (1H, dd, *J*= 6.3, 7.3 Hz), 6.04-5.92 (1H, m), 5.36-5.25 (2H, m), 4.75 (2H, d, *J*= 5.1 Hz), 4.21 (2H, q, *J*= 7.3 Hz), 4.17 (3H, s), 3.98 (3H, s), 1.36 (3H, t, *J*= 7.3 Hz); MS (FAB) *m*/*z* 556 ($M^+$).

B - 3 9 mp >300 °C (decomp.); IR (KBr) 1655, 1541, 1489, 1410, 1387, 1153 $cm^{-1}$; $^1H$ NMR (DMSO-$d_6$) δ 8.40 (1H, d, *J* = 1.2 Hz), 8.02 (1H, d, *J* = 8.8 Hz), 7.83 (1 H, dd, *J* = 1.2, 8.8 Hz), 6.05-5.90 (1H, m), 5.34 (1H, d, *J*= 17.1 Hz), 5.28 (1H, d, *J*= 10.3 Hz), 4.73 (2H, d, *J*= 5.4 Hz), 4.17 (2H, q, *J*= 7.3 Hz), 4.00 (3H, s), 3.96 (2H, t, *J*= 7.6 Hz), 3.48 (3H, s), 3.27 (2H, t, *J*= 7.6 Hz), 1.32 (3H, t, *J*= 7.3 Hz); MS (FAB) *m*/*z* 564 ($M^+$).

B - 4 0 mp 296-297 °C (decomp.); IR (KBr) 1636, 1543, 1487, 1435, 1375, 1151 $cm^{-1}$; $^1H$ NMR (DMSO-$d_6$) δ 8.44 (1H, d, *J*= 8.9 Hz), 8.23 (1H, d, *J*= 6.5 Hz), 8.02 (1H, d, *J*= 4.4 Hz), 7.78 (1H, dd, *J*= 7.0, 8.9 Hz), 7.74 (1H, d, *J*= 4.4 Hz), 7.03 (1H, dd, *J*= 6.5, 7.0 Hz), 4.25-4.00 (7H, m), 3.86 (3H, s), 1.41-1.20 (6H, m); MS (FAB) *m*/*z* 460 ($M^+$).

B - 4 1 mp>250°C(decomp.); IR (KBr) 1655, 1541, 1416, 1317, 1161 $cm^{-1}$; $^1H$ NMR (DMSO-$d_6$) δ. 8.05 (1H, d, *J* = 4.4 Hz), 7.82 (1H, d, *J* = 4.4 Hz), 5.99-5.86 (1H, m), 5.33-5.21 (2H, m), 4.65 (2H, d, *J*= 5.6 Hz), 4.13 (2H, q, *J*= 7.3 Hz), 3.94 (2H, t, *J*= 7.8 Hz), 3.87 (3H, s), 3.46 (3H, s), 3.26 (2H, t, *J*= 7.8 Hz), 1.29 (3H, t, *J*= 7.3 Hz); MS (FAB) *m*/*z* 480 ($M^+$).

C-2 mp.224-228°C(decomp.), 1H-NMR(DMSO-d6),δ8.40(br,1H),8.01(d,1H,*J*=8.3Hz),7.93(d,1H,*J*=7.8Hz),7.88(d,1H,*J* =13.2Hz),7.82(m,1H),7.53(t,1H,*J*=7.3Hz),7.37(t,1H,*J*=8.0Hz),6.18(d,1H,*J*=13.2Hz),4.09( q,2H,J=7.5Hz),4.02(s,3H),3.83(s,3H),1.31(t,3H,J=7.5Hz).

C-3 mp.205-210°C(decomp.), 1H-NMR(DMSO-d6),δ8.32(d,1H,*J*=6.1Hz),7.86(d,1H,*J*=7.8Hz),7.75(d,1H,*J*=7.8Hz),7.5 4-7.49(m,2H),7.41-7.38(m,2H),7.22-7.18(m,1H),5.56(d,1H,*J*=12.5Hz),4.02(s,3H),3.93(q ,2H,*J*=7.5Hz),3.53(s,3H),1.26(t,3H,*J*=7.5Hz).

C-4 mp.228-235°C(decomp.), 1H-NMR(DMSO-d6),δ7.91-7.89(m,2H),7.84(d, 1H,$J$=13.2Hz),7.70-7.6 1(m,5H),7.51 (t, 1 H,$J$=7.5Hz),7.34(t,1H,$J$=7.5Hz),6.14(d,1H,$J$=13.2Hz),4.04(q,2H,$J$=6.8Hz),3.79(s,3H),3.7 2(s,3H),1.28(t,3H, J=6.8Hz).

A-20 : mp 282-285 °C; IR (KBr) 1523, 1494, 1303 $cm^{-1}$; $^1H$ NMR (400 MHz, DMSO-$d_6$) δ 8.59 (d, 1H, $J$= 7.6 Hz), 8.44 (d, 1H, $J$= 6.4 Hz), 8.00 (d, 1H, $J$= 4.8 Hz), 7.72 (d, 1H, $J$= 4.8 Hz), 7.32 (t, 1H, $J$= 8.0 Hz), 4.20 (s, 3H), 4.09 (q, 2H, $J$= 6.8 Hz), 3.07 (s, 3H), 1.31 (t, 3H, $J$= 6.8 Hz); LRMS ($FAB^+$) *m/z:* 425 ($M^+$).

A-21 : orange crystals, mp 275-278 °C; IR (KBr) 1544, 1463, 1429, 1301 $cm^{-1}$; $^1H$ NMR (400 MHz, DMSO-$d_6$) δ 8.65 (d, 1H, $J$= 7.6 Hz), 8.48 (d, 1H, $J$= 6.4 Hz), 8.26 (d, 1H, $J$= 8.0 Hz), 7.95 (d, 1H, $J$= 8.4 Hz), 7.72 (t, 1H, $J$= 8.0 Hz), 7.56 (t, 1H, $J$= 8.0 Hz), 7.35 (t, 1H, $J$= 7.2 Hz), 4.22 (s, 3H), 4.18-4.15 (m, 2H), 4.01 (s, 3H), 1.35 (t, 3H, $J$= 7.2 Hz); LRMS ($FAB^+$) *m/z*: 440 ($M^+$).

A-22 : orange crystals, mp 275-277 °C; IR (KBr) 1533, 1490, 1305 $cm^{-1}$; $^1H$ NMR (400 MHz, DMSO-$d_6$) δ 8.73 (d, 1H, $J$= 6.0 Hz), 8.42 (d, 1H, $J$= 7.2 Hz), 8.34 (t, 1H, $J$= 8.0 Hz), 8.27 (d, 1H, $J$= 6.4 Hz), 7.88 (d, 1H, $J$= 8.4 Hz), 7.47 (t, 1H, $J$= 6.8 Hz), 7.16 (t, 1H, $J$= 6.8 Hz), 4.07 (s, 3H), 4.03 (s, 3H), 4.03-3.98 (m, 2H), 1.28 (t, 3H, $J$= 6.8 Hz); LRMS ($FAB^+$) *m/z:* 384 ($M^+$).

B-42 : mp >300 °C; IR (KBr) 1487, 1439 $cm^{-1}$; $^1H$ NMR (400 MHz, DMSO-$d_6$) δ 8.76 (d, 1H, $J$= 6.8 Hz), 8.40-8.36 (m, 2H), 8.25 (d, 1H, $J$= 6.8 Hz), 7.89 (d, 1H, $J$= 6.0 Hz), 7.53 (t, 1H, $J$= 7.2 Hz), 7.14 (t, 1H, $J$= 7.2 Hz), 4.13 (s, 3H), 4.04-4.00 (m, 7H), 1.30-1.23 (m, 6H); LRMS ($FAB^+$) *m/z:* 511 ($M^+$).

C-1 : green crystals, mp 172-174 °C; IR (KBr): 1521, 1193, 1130 $cm^{-1}$; $^1H$ NMR (300 MHz, DMSO·*d6)* δ 8.79 (d, 1H, $J$= 6.3 Hz), 8.40 (t, 1H, $J$= 7.6 Hz), 7.89 (d, 1H, $J$= 8.2 Hz), 7.78 (d, 1H, $J$= 7.6 Hz), 7.62-7.56 (m, 2H), 7.46-7.38 (m, 4H), 7.22 (t, 1H, $J$= 6.6 Hz), 7.10 (d, 1H, $J$= 8.0 Hz), 5.68 (d, 1H, $J$= 12.9 Hz), 4.04 (s, 3H), 3.93 (q, 1H, $J$= 7.7 Hz), 3.58 (s, 3H), 2.26 (s, 3H), 1.24 (t, 3H, $J$= 7.7 Hz).

C-7 : green crystals, mp 195-199 °C; IR (KBr) 1515, 1488, 1149, 1130 $cm^{-1}$; $^1H$ NMR (400 MHz, DMSO-$d_6$) δ 8.33 (d, 1H, $J$= 13.6 Hz), 8.01-7.81 (m, 4H), 7.76-7.66 (m, 4H), 7.61-7.41 (m, 3H), 5.86 (d, 1H, $J$= 13.6 Hz), 4.10-4.04 (m, 2H), 3.91 (s, 3H), 3.72 (s, 3H), 1.29 (t, 3H, $J$= 6.8 Hz); LRMS ($FAB^+$) *m/z*: 520 ($M^+$).

C-8 : green crystals, mp 239-241 °C; IR (KBr): 1525, 1355, 1274 $cm^{-1}$; $^1H$ NMR (400 MHz, DMSO-$d_6$) δ 8.27 (d, 1H, $J$ = 13.6 Hz), 8.05 (d, 1H, $J$ = 4.4 Hz), 7.98-7.77 (m, 5H), 7.70-7.66 (br, 1H), 7.40 (q, 1H, $J$= 7.2 Hz), 5.77 (d, 1H, $J$= 13.6 Hz), 4.04-4.01 (br, 2H), 3.88-3.87 (br, 6H), 1.27 (t, 3H, $J$= 7.2 Hz); LRMS ($FAB^+$) *m/z*: 409 ($M^+$).

C-9 : green crystals, mp 251-253 °C; IR (KBr): 1515, 1355, 1261, 1224 $cm^{-1}$; $^1H$ NMR (400 MHz, DMSO-$d_6$) δ 8.31 (d, 1H, $J$= 13.6 Hz), 8.24 (d, 1H, $J$= 8.4 Hz), 7.99-7.94 (m, 3H), 7.87 (d, 1H, $J$= 8.4 Hz), 7.82 (d, 1H, $J$= 7.6 Hz), 7.73-7.69 (br, 2H), 7.59-7.41 (m, 2H), 5.87 (d, 1H, $J$= 13.6 Hz), 4.13-4.08 (br, 2H), 4.03 (s, 3H), 3.92 (s, 3H), 1.32 (t, 3H, $J$ = 6.8 Hz); LRMS ($FAB^+$) *m/z*: 459 ($M^+$).

C-10 : purple crystals, mp 253-256 °C; IR (KBr): 1508, 1361, 1317, 1191 $cm^{-1}$; $^1H$ NMR (400 MHz, DMSO-$d_6$) δ 8.46 (br, 1H), 7.88-7.78 (m, 3H), 7.59 (d, 1H, $J$= 8.8 Hz), 7.49 (t, 1H, $J$= 7.6 Hz), 7.31 (t, 1H, $J$= 7.6 Hz), 6.08 (d, 1H, $J$= 12.4 Hz), 4.04-4.00 (br, 2H), 3.89 (s, 3H), 3.77 (s, 3H), 1.28-1.25 (br, 3H); LRMS ($FAB^+$) *mlz.* 415 ($M^+$).

C-11 : green crystals, mp 245-247 °C; IR (KBr): 1496, 1458, 1359 $cm^{-1}$; $^1H$ NMR (400 MHz, DMSO-$d_6$) δ 8.27 (d, 1H, $J$= 8.0 Hz), 8.03-7.87 (m, 3H), 7.78 (t, 1H, $J$= 8.0 Hz), 7.66-7.61 (m, 2H), 7.53 (t, 1H, $J$= 8.0 Hz), 7.37 (t, 1H, $J$= 7.6 Hz), 6.17 (d, 1H, $J$= 13.2 Hz), 4.11 (q, 1H, $J$= 7.2 Hz), 4.05 (s, 3H), 3.82 (s, 3H), 1.32 (t, 3H, $J$= 7.2 Hz); LRMS ($FAB^+$) *m/z:* 465 ($M^+$).

C-12 : green crystals, mp 236-239 °C; IR (KBr): 1506, 1357, 1259, 1149, 1132 $cm^{-1}$; $^1H$ NMR (400 MHz, DMSO-$d_6$) δ 9.14 (s, 1H), 8.51 (d, 1H, $J$= 9.2 Hz), 8.15 (d, 1H, $J$= 13.2 Hz), 7.91 (d, 1H, $J$= 9.2 Hz), 7.84 (d, 1H, $J$= 9.2 Hz), 7.70-7.68 (m, 3H), 7.61 (t, 1H, $J$= 7.6 Hz), 7.30 (t, 1H, $J$= 7.2 Hz), 5.42 (d, 1H, $J$= 12.8 Hz), 4.02 (s, 3H), 3.95 (q, 2H, $J$= 7.2 Hz), 3.68 (s, 3H), 1.25 (t, 3H, $J$= 7.2 Hz) ; LRMS ($FAB^+$) *m/z*: 437 ($M^+$).

D-1 : purple crystals, mp 298-300 °C; IR (KBr): 1552, 1494, 1369, 1261, 1136 $cm^{-1}$; $^1H$ NMR (400 MHz, DMSO-$d_6$) δ 8.78 (d, 1H, $J$ = 8.0 Hz), 8.40 (t, 1H, $J$= 7.6 Hz), 8.02-7.97 (m, 1H), 7.90 (d, 1H, $J$= 8.4 Hz), 7.74-7.51 (m, 6H), 7.28 (t, 1H, $J$= 7.2 Hz), 5.47 (d, 1H, $J$ = 13.2 Hz), 4.05 (s, 3H), 4.02-3.98 (m, 2H), 3.72 (s, 3H), 3.62-3.58 (m, 2H), 1.29 (t, 3H, $J$= 7.2 Hz), 1.22 (t, 3H, $J$= 7.2 Hz); LRMS ($FAB^+$) *m/z*: 530 ($M^+$).

D-2 : green crystals, mp >300 °C; IR (KBr): 1496, 1369, 1149 $cm^{-1}$; $^{1}H$ NMR (400 MHz, DMSO-$d_6$) δ 8.78 (d, 1H, *J*= 5.0 Hz), 8.41 (t, 1H, *J*= 7.2 Hz), 7.88 (d, 1H, *J*= 8.4 Hz), 7.77 (d, 1H, *J*= 7.9 Hz), 7.58 (t, 1H, *J*= 6.5 Hz), 7.48-7.42 (m, 3H), 7.22 (t, 1H, *J*= 6.8 Hz), 5.75 (d, 1H, *J*= 13.2 Hz), 4.05 (s, 3H), 4.02-3.97 (m, 2H), 3.66 (s, 3H), 1.28 (t, 3H, *J* = 6.8 Hz), 1.22 (t, 3H, *J*= 7.6 Hz); LRMS ($FAB^+$) *m/z:* 536 ($M^+$).

Improved therapeutic/prophylactic agent can be provided by having the compound of the invention as an active ingredient.

This application claims a priority based on the Japanese Patent Application No.2005-184183 filed on 24 June 2005, and its disclosure including specification and claims is incorporated herein by reference in its entirety.

## Claims

1. Pharmaceutical composition containing at least one of azarhodacyanine compounds represented by general formula (1) as an active ingredient:
(Formula 1)
wherein , R1 and R4 respectively represents alkyl group which may be same or different from each other, R2 and R3 respectively represents alkyl, aryl, or heterocyclic group, X1 and X2 respectively represents S, O, Se, -CR5R6- (R5 and R6 respectively represents alkyl group), -NR7- (R7 represents alkyl, aryl, or heterocyclic group), or -CR8=CR9- (R8 and R9 represents hydrogen or substituent, alternatively, R8 and R9 may bind together to form alicyclic ring, aromatic ring, or heterocyclic ring) , Y1 and Y2 respectively represents S, O, Se, or -NR8- (R8 is a alkyl, aryl, or heterocyclic group), Z1 and Z2 respectively represents an atomic group necessary for forming a five member ring or a six member ring, L1 and L2 respectively represents methine group, wherein when p>0 and L1 is a substituted methine group, L1 and R1 may bind together to form a five member ring or a six member ring, Q represents physiologically acceptable anion, k represents one integer from 0 to 2 necessary to null whole molecular charge, m and n respectively represents 0 or 1 which may be same or different from each other, p represents one integer from 0 to 2, and q represents one integer from 0 to 2.

2. Pharmaceutical composition according to claim 1, wherein the composition is used as a therapeutic and/or prophylactic agent against protozoa infection.

3. Pharmaceutical composition according to claim 2, wherein the protozoa infection is one of malaria, leishmania, African sleeping sickness, Chagas disease, toxoplasmosis, lymphatic filariasis, babesiosis, or coccidiosis.

4. Pharmaceutical composition according to claim 3, wherein the composition is used as a therapeutic and/or prophylactic agent against malaria.

5. Pharmaceutical composition containing at least one of compounds represented by general formula (2) as an active ingredient:
(Formula 2)
wherein R1 and R3 respectively represents alkyl group which may be same or different from each other, R2 represents alkyl, aryl, or heterocyclic group, X1 and X2 respectively represents S, O, Se, -CR4R5- (R4 and R5 respectively represents alkyl group), -NR6- (R6 represents alkyl, aryl, or heterocyclic group), or -CR7=CR8- (R7 and R8 represents hydrogen or substituent, alternatively, R7 and R8 may bind together to form alicyclic ring, aromatic ring, or heterocyclic ring), Z1 and Z2 respectively represents atomic group necessary for forming a five member ring or a six member ring, Q represents physiologically acceptable anion, k represents one integer from 0 to 2 necessary to null whole molecular charge, and m and n respectively represents 0 or 1 which may be same or different from each other.

6. Pharmaceutical composition according to claim 5, wherein R1 and R3 are methyl group, and R2 is ethyl group or phenyl group.

7. Pharmaceutical composition according to claim 5 or 6, wherein the composition is used as a therapeutic and/or prophylactic agent against protozoa infection.

8. Conjugated compound represented by general formula (3):
(Formula 3)
wherein, R1 and R4 respectively represents alkyl group which may be same or different from each other, R2 and R3 respectively represents alkyl, aryl, or heterocyclic group, X1 and X2 respectively represents S, O, Se, -CR5R6-

(R5 and R6 respectively represents alkyl group), -NR7- (R7 represents alkyl, aryl, or heterocyclic group), or -CR8=CR9- (R8 and R9 represents hydrogen or substituent, alternatively, R8 and R9 may bind together to form alicyclic ring, aromatic ring, or heterocyclic ring), Z1 and Z2 respectively represents atomic group necessary for forming a five member ring or a six member ring, Q represents physiologically acceptable anion, k represents one integer from 0 to 2 necessary to null whole molecular charge, and m and n respectively represents 0 or 1 which may be same or different from each other.

**9.** A method to manufacturing the conjugated compound represented by general formula (3), the compound having a thioiminium compound represented by general formula (4) and a nitrogen containing compound represented by general formula (5) as material,
(Formula 4)
wherein, R1 and R10 respectively represents alkyl group which may be same or different from each other, R2 represents alkyl, aryl, or heterocyclic group, X1 represents S, O, Se, -CR5R6-(R5 and R6 respectively represents alkyl group), -NR7- (R7 represents alkyl, aryl, or heterocyclic group), or -CR8=CR9-(R8 and R9 represents hydrogen or substituent, alternatively, R8 and R9 may bind together to form an alicyclic ring, aromatic ring, or heterocyclic ring), Z1 represents an atomic group necessary for forming a five member ring or a six member ring, Q represents physiologically acceptable anion, k represents one integer from 0 to 2 necessary to null whole molecular charge, and m represents 0 or 1,
(Formula 5)
wherein, R4 represents alkyl group, R3 represents alkyl, aryl, or heterocyclic group, X2 represents S, O, Se, -CR5R6-(R5 and R6 respectively represents alkyl group), -NR7- (R7 represents alkyl, aryl, or heterocyclic group), or -CR8=CR9- (R8 and R9 represents hydrogen or substituent, alternatively, R8 and R9 may bind together to form alicyclic ring, aromatic ring, or heterocyclic ring), Z2 represents an atomic group necessary for forming a five member ring or a six member ring, Q represents physiologically acceptable anion, k represents one integer from 0 to 2 necessary to null whole molecular charge, and n represents 0 or 1.

**10.** Pharmaceutical composition containing at least one compound represented by general formula (3) as an active ingredient.

**11.** Pharmaceutical composition according to Claim 10, wherein R1 and R4 are methyl group.

**12.** Pharmaceutical composition according to claim 10 or 11, wherein the composition is used as a therapeutic and/or prophylactic agent against protozoa infection.

**13.** Pharmaceutical composition containing at least one compound represented by general formula (6) as an active ingredient,
(Formula 6)
wherein , R1 and R3 respectively represents alkyl group which may be same or different from each other, R2 represents alkyl, aryl, or heterocyclic group, X1 and X2 respectively represents S, O, Se, -CR4R5- (R4 and R5 respectively represents alkyl group), -NR6- (R6 represents alkyl, aryl, or heterocyclic group), or -CR7=CR8- (R7 and R8 represent hydrogen or substituent, alternatively, R7 and R8 may bind together to form alicyclic ring, aromatic ring, or heterocyclic ring), Z1 and Z2 respectively represents an atomic group necessary for forming a five member ring or a six member ring, Q represents physiologically acceptable anion, k represents one integer from 0 to 2 necessary to null whole molecular charge, and m and n represent 0 or 1 which be may same or different from each other.

**14.** Pharmaceutical composition according to claim 13 wherein R1 and R3 are methyl group or ethyl group, and R2 is ethyl group.

**15.** Pharmaceutical composition according to claim 13 or 14, wherein the composition is used as a therapeutic and/or prophylactic agent against protozoa infection.

**16.** Conjugated compound represented by general formula (7):
(Formula 7)
wherein, R1 and R4 respectively represents alkyl group which may be same or different from each other, R2 and R3 respectively represents alkyl, aryl, or heterocyclic group, X1 and X2 respectively represents S, O, Se, -CR5R6- (R5 and R6 respectively represents alkyl group), -NR7- (R7 represents alkyl, aryl, or heterocyclic group), or -CR8=CR9- (R8 and R9 represents hydrogen or substituent, alternatively, R8 and R9 may bind together to form

alicyclic ring, aromatic ring, or heterocyclic ring), Z1 and Z2 respectively represents atomic group necessary for forming a five member ring or a six member ring, Q represents physiologically acceptable anion, k represents one integer from 0 to 2 necessary to null whole molecular charge, and m and n respectively represents 0 or 1 which may be same or different from each other.

**17.** A method to manufacturing the conjugated compound represented by general formula (7), the compound having a thioiminium compound represented by general formula (8) and a nitrogen containing compound represented by general formula (5) as material,
(Formula 8) wherein, R1 and R10 respectively represents alkyl group which may be same or different from each other, R2 represents alkyl, aryl, or heterocyclic group, X1 represents S, O, Se, -CR5R6-(R5 and R6 respectively represents alkyl group), -NR7- (R7 represents alkyl, aryl, or heterocyclic group), or -CR8=CR9-(R8 and R9 represents hydrogen or substituent, alternatively, R8 and R9 may bind together to form an alicyclic ring, aromatic ring, or heterocyclic ring), Z1 represents an atomic group necessary for forming a five member ring or a six member ring, Q represents physiologically acceptable anion, k represents one integer from 0 to 2 necessary to null whole molecular charge, and m represents 0 or 1,
(Formula 5)
wherein, R4 represents alkyl group, R3 represents alkyl, aryl, or heterocyclic group, X2 represents S, O, Se, -CR5R6- (R5 and R6 respectively represents alkyl group), -NR7- (R7 represents alkyl, aryl, or heterocyclic group), or -CR8=CR9- (R8 and R9 represents hydrogen or substituent, alternatively, R8 and R9 may bind together to form alicyclic ring, aromatic ring, or heterocyclic ring), Z2 represents an atomic group necessary for forming a five member ring or a six member ring, Q represents physiologically acceptable anion, k represents one integer from 0 to 2 necessary to null whole molecular charge, and n represents 0 or 1.

**18.** Pharmaceutical composition containing at least one compound represented by general formula (7) as an active ingredient.

**19.** Pharmaceutical composition according to Claim 18, wherein R1 and R4 are methyl group.

**20.** Pharmaceutical composition according to claim 18 or 19, wherein the composition is used as a therapeutic and/or prophylactic agent against protozoa infection.
Formula:

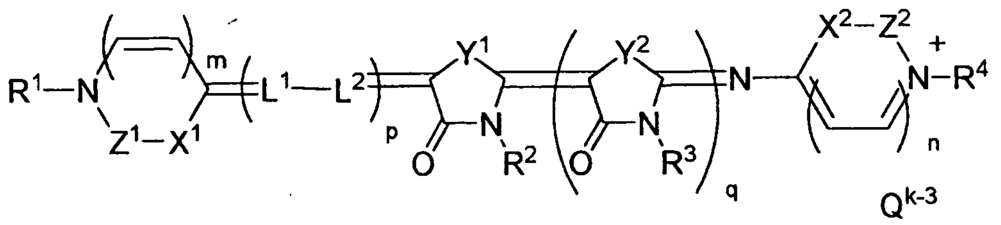

(1)

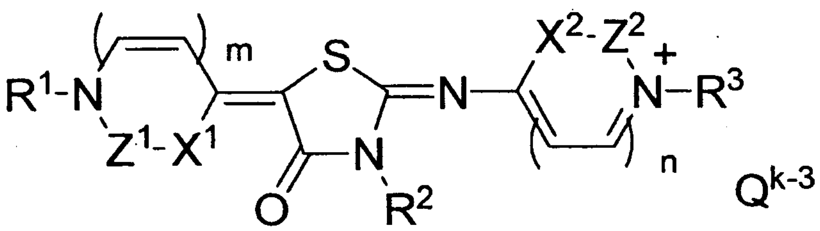

(2)

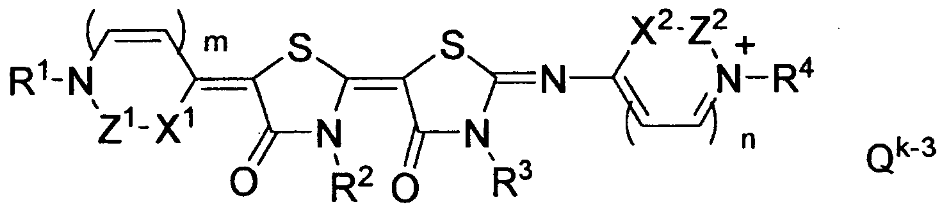

(3)

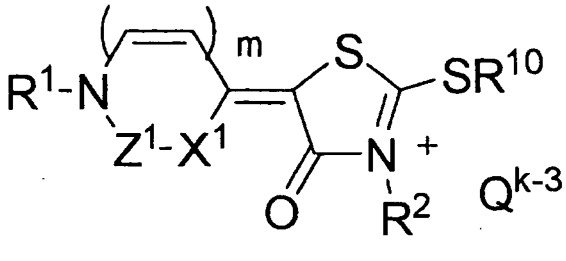

(4)

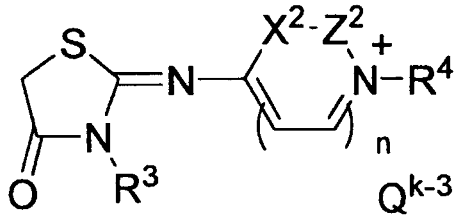

(5)

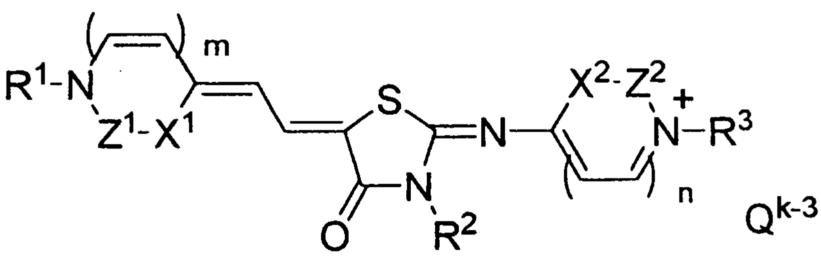

(6)

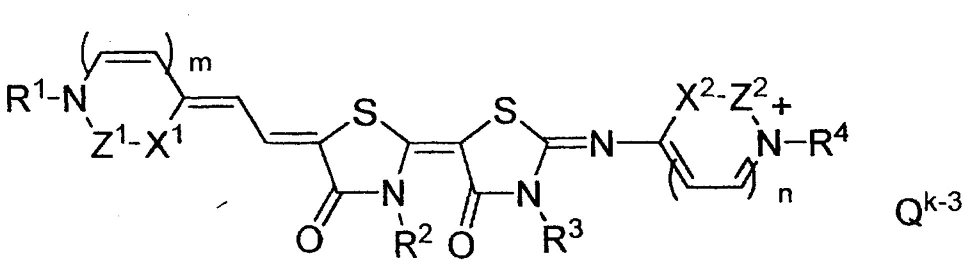

(7)

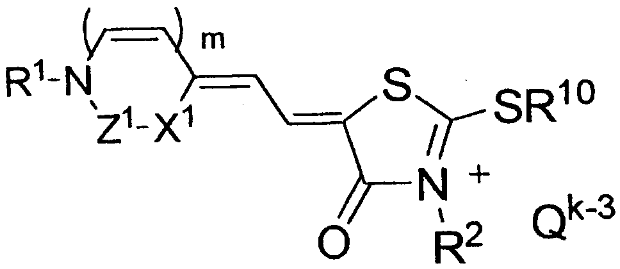

(8)

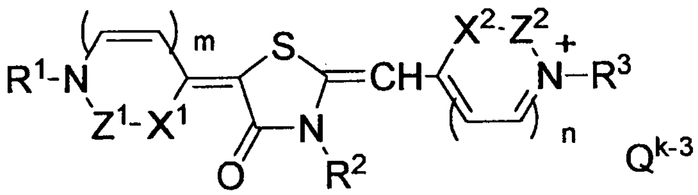

(9)

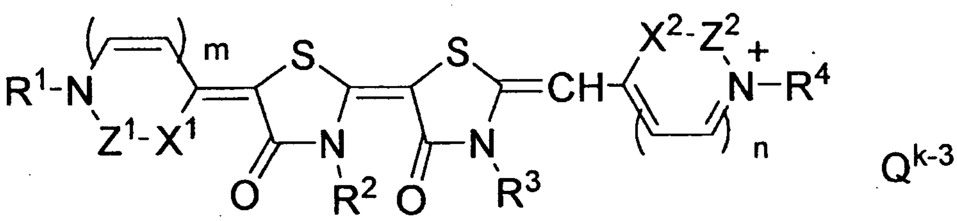

(10)

## INTERNATIONAL SEARCH REPORT

International application No.
PCT/JP2006/311198

A. CLASSIFICATION OF SUBJECT MATTER
See extra sheet.

According to International Patent Classification (IPC) or to both national classification and IPC

B. FIELDS SEARCHED

Minimum documentation searched (classification system followed by classification symbols)
C07D277/20, A61K31/426, A61K31/428, A61K31/437, A61K31/4439, A61K31/444, A61K31/4709, A61P33/02, A61P33/06, C07D277/38, C07D277/82, C07D417/14, C07D498/04, C07D513/04

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2006 |
| Kokai Jitsuyo Shinan Koho | 1971-2006 | Toroku Jitsuyo Shinan Koho | 1994-2006 |

Electronic data base consulted during the international search (name of data base and, where practicable, search terms used)
CAplus(STN), REGISTRY(STN), WPI(DIALOG)

C. DOCUMENTS CONSIDERED TO BE RELEVANT

| Category* | Citation of document, with indication, where appropriate, of the relevant passages | Relevant to claim No. |
|---|---|---|
| X<br>Y | US 5618831 A (Fuji Photo Film Co., Ltd.),<br>08 April, 1997 (08.04.97),<br>Claims 1 to 4, 5; compound I-10<br>& JP 6-220053 A & US 547645 A | 1,5,6,13,14<br>2-4,7,15 |
| X<br>Y | JP 6-293642 A (Fuji Photo Film Co., Ltd.),<br>21 October, 1994 (21.10.94),<br>Claim 1; compound No.56<br>& US 5670530 A & JP 2879395 B2 | 1,5,6,13,14<br>2-4,7,15 |
| X<br>Y | JP 6-234639 A (Fuji Photo Film Co., Ltd.),<br>23 August, 1994 (23.08.94),<br>Claim 1; compound Nos. 38, 39<br>(Family: none) | 1,5,6,13,14<br>2-4,7,15 |

☒ Further documents are listed in the continuation of Box C.  ☐ See patent family annex.

* Special categories of cited documents:
- "A" document defining the general state of the art which is not considered to be of particular relevance
- "E" earlier application or patent but published on or after the international filing date
- "L" document which may throw doubts on priority claim(s) or which is cited to establish the publication date of another citation or other special reason (as specified)
- "O" document referring to an oral disclosure, use, exhibition or other means
- "P" document published prior to the international filing date but later than the priority date claimed
- "T" later document published after the international filing date or priority date and not in conflict with the application but cited to understand the principle or theory underlying the invention
- "X" document of particular relevance; the claimed invention cannot be considered novel or cannot be considered to involve an inventive step when the document is taken alone
- "Y" document of particular relevance; the claimed invention cannot be considered to involve an inventive step when the document is combined with one or more other such documents, such combination being obvious to a person skilled in the art
- "&" document member of the same patent family

| Date of the actual completion of the international search | Date of mailing of the international search report |
|---|---|
| 11 August, 2006 (11.08.06) | 22 August, 2006 (22.08.06) |
| Name and mailing address of the ISA/<br>Japanese Patent Office<br>Facsimile No. | Authorized officer<br><br>Telephone No. |

Form PCT/ISA/210 (second sheet) (April 2005)

**INTERNATIONAL SEARCH REPORT**

International application No.
PCT/JP2006/311198

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2000-191531 A (Fuji Photo Film Co., Ltd.), 11 July, 2000 (11.07.00), Full text (Family: none) | 1-7,13-15 |
| Y | JP 2003-34641 A (Japan Science and Technology Corp.), 07 February, 2003 (07.02.03), Full text & WO 2003/007948 A1 & AU 2002313885 A1 | 1-7,13-15 |
| Y | JP 2003-34642 A (Japan Science and Technology Corp.), 07 February, 2003 (07.02.03), Full text (Family: none) | 1-7,13-15 |
| Y | JP 2004-331545 A (Japan Science and Technology Agency), 25 November, 2004 (25.11.04), Full text & WO 2004/108695 A1 & EP 1623981 A1 & BR 200409995 A | 1-7,13-15 |
| X<br>A | JP 8-59467 A (Fuji Photo Film Co., Ltd.), 05 March, 1996 (05.03.96), Claims 1 to 9 (Family: none) | 1,5,6<br>2-4,7,13-15 |
| X<br>A | JP 10-506878 A (Fuji Photo Film Co., Ltd.), 07 July, 1998 (07.07.98), Claims 10 to 15 & WO 96/03393 A1 & AU 9529366 A & US 5599825 A & EP 772607 A1 & AU 678116 B | 1,5,6,13,14<br>2-4,7,15 |
| P,X<br>P,A | US 2006/0035926 A1 (Shiow-Ju Lee), 16 February, 2006 (16.02.06), Compound No.197 & EP 1627635 A2 | 1,13,14<br>2-7,15 |
| P,Y | JP 2006-104115 A (Japan Science and Technology Agency), 20 April, 2006 (20.04.06), Full text & WO 2006/38513 A1 | 1-7,13-15 |
| P,Y | JP 2006-104116 A (Japan Science and Technology Agency), 20 April, 2006 (20.04.06), Full text & WO 2006/38550 A1 | 1-7,13-15 |

Form PCT/ISA/210 (continuation of second sheet) (April 2005)

**INTERNATIONAL SEARCH REPORT**

International application No.
PCT/JP2006/311198

Continuation of A. CLASSIFICATION OF SUBJECT MATTER
(International Patent Classification (IPC))

*C07D277/20*(2006.01)i, *A61K31/426*(2006.01)i, *A61K31/428*(2006.01)i, *A61K31/437*(2006.01)i, *A61K31/4439*(2006.01)i, *A61K31/444*(2006.01)i, *A61K31/4709*(2006.01)i, *A61P33/02*(2006.01)i, *A61P33/06*(2006.01)i, *C07D277/38*(2006.01)i, *C07D277/82*(2006.01)i, *C07D417/14*(2006.01)i, *C07D498/04*(2006.01)i, *C07D513/04*(2006.01)i

(According to International Patent Classification (IPC) or to both national classification and IPC)

Form PCT/ISA/210 (extra sheet) (April 2005)

**INTERNATIONAL SEARCH REPORT**

International application No.
PCT/JP2006/311198

**Box No. II Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III Observations where unity of invention is lacking (Continuation of item 3 of first sheet)**

This International Searching Authority found multiple inventions in this international application, as follows:
See extra sheet.

1. ☐ As all required additional search fees were timely paid by the applicant, this international search report covers all searchable claims.
2. ☐ As all searchable claims could be searched without effort justifying an additional fee, this Authority did not invite payment of any additional fee.
3. ☐ As only some of the required additional search fees were timely paid by the applicant, this international search report covers only those claims for which fees were paid, specifically claims Nos.:

4. ☒ No required additional search fees were timely paid by the applicant. Consequently, this international search report is restricted to the invention first mentioned in the claims; it is covered by claims Nos.:
Parts of 1-4 referring to the general formulae (2) and (6) and 5-7 and 13-15.

**Remark on Protest** the

☐ The additional search fees were accompanied by the applicant's protest and, where applicable, payment of a protest fee..

☐ The additional search fees were accompanied by the applicant's protest but the applicable protest fee was not paid within the time limit specified in the invitation.

☒ No protest accompanied the payment of additional search fees.

Form PCT/ISA/210 (continuation of first sheet (2)) (April 2005)

**INTERNATIONAL SEARCH REPORT**

International application No.
PCT/JP2006/311198

Continuation of Box No.III of continuation of first sheet(2)

Claims 1-20

These claims include the following inventions:

(1) a pharmaceutical composition comprising at least one of the azarhodacyanine compounds of the general formula (1) including the general formulae (2), (3), (6) and (7) as an active ingredient (claims 1-4);

(2) a pharmaceutical composition comprising at least one of the compounds represented by the general formula (2) as an active ingredient (claims 8-12);

(3) a conjugated compound represented by the general formula (3), a process for producing the compound, and a pharmaceutical composition comprising at least one of the compounds as an active ingredient (claims 8-12);

(4) a pharmaceutical composition comprising at least one of the compounds represented by the general formula (6) as an active ingredient (claims 13-15); and

(5) a conjugated compound represented by the general formula (7), a process for producing the compound, and a pharmaceutical composition comprising at least one of the compounds as an active ingredient (claims 16-20).

The chemical structure common to the compounds included in the inventions of the claims is only the following moiety.

[Chemical formula]

However, as disclosed in US 5618831 A, compounds having this chemical structure and pharmaceutical composition using the compounds are known. Therefore, it cannot be considered that the chemical structure is a special technical feature common to these inventions.

Thus, these inventions cannot be regarded as a group of inventions so linked as to form a single general inventive concept, and the present invention includes the following different four groups of inventions that do not comply with the requirement of unity of invention:

a) inventions of parts of claims 1-4 referring to the general formula (2) and inventions of claims 5-7;

b) inventions of parts of claims 1-4 referring to the general formula (3) and inventions of claims 8-12;

c) inventions of parts of claims 1-4 referring to the general formula (6) and inventions of claims 13-15; and

d) inventions of parts of claims 1-4 referring to the general formula (7) and inventions of claims 16-20.

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP 6220053 A
- JP 6293642 A
- JP 2000191531 A
- JP 2003034641 A
- JP 2003034642 A
- JP 2004331545 A
- JP 2003034640 A
- JP 2005184183 A